# EUROPEAN PATENT APPLICATION

(11) **EP 4 707 293 A1**
(43) Date of publication of application: **11.03.2026**
(21) Application number: 24799876.8
(22) Date of filing: 26.04.2024
(51) Int. Cl.: C07K 14/135, C12N 15/45, A61K 39/155, A61P 31/14

(54) **MUTANT OF RSV F PROTEIN**

(30) Priority: 04.05.2023 CN 202310489328
(71) Applicant: National Vaccine and Serum Institute (NVSI), Beijing 101111 (CN)
(72) Inventor: LI, Qiming, Beijing 101111 (CN); SU, Jiguo, Beijing 101111 (CN); ZHANG, Jing, Beijing 101111 (CN); LIANG, Yu, Beijing 101111 (CN); SHAO, Shuai, Beijing 101111 (CN); JIN, Yuqin, Beijing 101111 (CN); ZHANG, Xuefeng, Beijing 101111 (CN); HOU, Junwei, Beijing 101111 (CN); HAN, Zibo, Beijing 101111 (CN); ZHANG, Hao, Beijing 101111 (CN); LI, Xinyu, Beijing 101111 (CN); ZHAO, Zixin, Beijing 101111 (CN); DU, Lifang, Beijing 101111 (CN); HOU, Yanan, Beijing 101111 (CN); LEI, Zehua, Beijing 101111 (CN); LIU, Zhaoming, Beijing 101111 (CN); MA, Zhijing, Beijing 101111 (CN); TANG, Fang, Beijing 101111 (CN); CHEN, Shi, Beijing 101111 (CN); ZHENG, Fan, Beijing 101111 (CN); LIU, Ning, Beijing 101111 (CN); SHEN, Fujie, Beijing 101111 (CN); WU, Jinjuan, Beijing 101111 (CN); ZHENG, Xiang, Beijing 101111 (CN)
(74) Representative: Cabinet Chaillot
(86) International application number: PCT/CN2024/089988
(87) International publication number: WO 2024/227418

(57) **Abstract**

Disclosed is a mutant of a wild-type RSV F protein. The binding experiment of a pre-fusion conformation trimer-specific monoclonal antibody AM14 shows that the mutant provided in embodiments can have strong activity of binding to the AM14 monoclonal antibody, while the wild-type RSV F protein before artificial mutation has no activity of binding to the AM14 monoclonal antibody. The accelerated stability test result shows that the activity of binding of the mutant provided in the embodiments when placed in a 37°C environment for four weeks to the AM14 monoclonal antibody does not change obviously. The animal immune experiment result shows that compared with the wild-type RSV F protein before artificial mutation, the mutant provided in the embodiments can induce production of a neutralizing antibody having a higher titer.

## Description

### CROSS-REFERENCE TO RELATED APPLICATIONS

The present application claims the priority of Chinese Patent Application No. 202310489328.3, filed with the Chinese Patent Office on May 4, 2023 and entitled "RSV F PROTEIN MUTANTS", which is incorporated in its entirety herein by reference.

### TECHNICAL FIELD

The present disclosure relates to the field of bio-pharmaceuticals, in particular to a mutant of a respiratory syncytial virus (RSV) fusion (F) protein, a method for stabilizing a prefusion RSV F protein trimer, a vaccine, and other products.

### BACKGROUND OF THE INVENTION

Respiratory syncytial virus (RSV) is a common virus that causes infections of lungs and respiratory tracts. It leads to over 30 million cases of lower respiratory tract infections globally each year, with approximately 3 million patients requiring hospitalization. The RSV poses significant risks to infants, young children, and the elderly, potentially causing severe infections. It is the second leading cause of death in the infants and the young children and the major contributor to respiratory illness onset and death in the elderly. Currently, no vaccine has been approved for prevention of an RSV infection.

The RSV is an enveloped, negative-sense single-stranded RNA virus belonging to the genus *Orthopneumovirus* within the family *Pneumoviridae.* Its genome encodes 11 proteins, including 9 structural proteins and 2 nonstructural proteins. Two transmembrane glycoproteins, that is, attachment protein G and fusion protein F, on a virus particle surface play critical roles in an initial stage of viral infection and serve as primary targets for neutralizing antibodies. The RSV includes two major subtypes: subtype A and subtype B. As for the two subtypes, the G protein exhibits a significant divergence, and the F protein shows higher conservation, with a sequence homology exceeding 90% (Ref: Harshbarger W, et al. PLoS Pathog., 2020, 16:e1008943.).

The RSV F protein is a class I viral fusion protein responsible for the fusion between viruses and host cell membranes. The F protein is first translated into a single-chain inactive precursor protein F0 consisting of 574 amino acids in vivo. A signal peptide consisting of amino acids at positions 1 to 25 at an N-terminus of the F0 protein is removed by a signal peptidase. Furthermore, there are two furin cleavage sites at amino acids at positions 109/110 and 136/137 of the F0. Through the protease cleavage, a polypeptide with amino acids at positions 110 to 136 (referred to as p27) is removed, and an F1 consisting of amino acids at positions 137 to 574 and an F2 consisting of amino acids at positions 26 to 109 are generated. The F1 and the F2 are linked together by two disulfide bonds, forming an F protein monomer. In a natural state, three F protein monomers are further assembled to form a homotrimer, which then exerts its biological activity.

The RSV F protein exhibits two conformational states, namely a prefusion conformation and a postfusion conformation, and their spatial structures have been resolved (Ref: McLellan J. S., et al. Science, 2013, 340:1113-1117. McLellan J. S., et al. J. Virol., 2011, 85:7788-7796.). A prefusion trimer resembles a lollipop, which can be divided into a head domain far from a viral envelope and a tail domain proximal to the envelope. During viral infection of a host cell, refolding region 1 (RR1) at the head of the F protein and refolding region 2 (RR2) proximal to the envelope undergo significant conformational changes. RR1 transitions from a helical, randomly coiled, and β-sheet structure into a long helical structure and extends above the head domain. RR2 flips from the position proximal to the envelope to a position above the head domain far from the envelope and binds to RR1 to form a six-helix bundle. In this case, the F protein trimer transitions from a lollipop-shaped prefusion conformation to a cone-shaped postfusion conformation, causing the fusion between the viruses and the host cell membranes (Ref: Krarup A., et al. Nat. Commun., 2015, 6:8143).

Since the F protein is important in early stages of the RSV infection and is the primary target for neutralizing antibodies in human bodies, the F protein is one of the primary target antigens for vaccine development. However, most of highly active neutralizing antibodies produced after humans get infected target the prefusion conformation (Ref: Battles M. B., McLellan J. S. Nat. Rev. Microbiol., 2019, 17:233-245). Numerous studies have successfully isolated high-affinity neutralizing antibodies that specifically bind to the prefusion conformation of the F protein, such as the AM14 monoclonal antibody, which targets an interface between different monomers of the prefusion trimer (Ref: Gilman M. S. A. et al. PLoS Pathog., 2015, 11:e1005035.). However, the prefusion conformation of the F protein is a metastable state that easily undergoes spontaneous conversion to the postfusion conformation. Animal immunological experiments have shown that the neutralizing antibody induced by the F protein in the prefusion conformation has a much higher titer than that induced by the F protein in the postfusion conformation (Ref: Battles M. B., McLellan J. S. Nat. Rev. Microbiol., 2019, 17:233-245 ; Krarup A. et al. Nat. Commun., 2015, 6:8143). Accordingly, stabilizing the RSV F protein in a prefusion trimer state and preventing its transition to the postfusion conformation can make the RSV F protein an optimal antigen for vaccine development, providing an effective strategy for RSV vaccine research and development.

However, to date, the development of vaccines targeting the prefusion RSV F protein trimer has not been able to satisfy widespread clinical needs. Thus, it has become urgent to find a new mutant of the RSV F protein that stabilizes the RSV F protein in the prefusion trimer state and can induce an excellent neutralizing antibody titer.

### BRIEF SUMMARY OF THE INVENTION

Technical problem to be solved:
In an aspect of the present disclosure, as for a lack of a novel mutant, that can stably maintain a prefusion trimer state and induce an excellent neutralizing antibody titer, of a respiratory syncytial virus (RSV) fusion (F) protein in the prior art, a novel mutant of the RSV F protein and a novel strategy for stabilizing a prefusion RSV F protein trimer are provided.

Specifically, in embodiments of the present disclosure, through structure alignment of a prefusion conformation and a postfusion conformation of the RSV F protein, other conformational change regions coupled with a refolding region 1 (RR1) conformational transition are identified in a head domain of the RSV F protein, and mutation is introduced into these regions to stabilize the conformation. The RR1 conformational transition is prevented through coupling regulation, and/or the conformational transition is prevented by directly introducing mutation into interaction regions involved in RR1. Based on the strategy, various mutation solutions are provided in the embodiments of the present disclosure to stabilize the RSV F protein in the prefusion trimer state. Meanwhile, in the embodiments of the present disclosure, mutation is introduced into a tail domain to further enhance stability of the prefusion RSV F protein trimer, so as to solve the above problems.

Technical solutions provided in the present disclosure:
A mutant of a wild-type RSV F protein is provided. Compared with a wild-type RSV F protein before artificial mutation, the mutant has at least one amino acid mutation site. The amino acid mutation site includes:
(a) any one of amino acids at positions 73 to 96; or
(b) any one of amino acids at positions 216 to 240; or
(c) an amino acid at position 141, an amino acid at position 190, an amino acid at position 193, an amino acid at position 195, an amino acid at position 199, or an amino acid at position 373.

In the embodiments of the present disclosure, based on spatial structure alignment analysis of the prefusion conformation and the postfusion conformation of the RSV F protein, the mutation is introduced into the head domain to prevent the RR1 conformational transition, and the F protein is stabilized in the prefusion trimer state. A binding experiment of a prefusion trimer-specific monoclonal antibody AM14 shows that the mutant provided in the embodiments of the present disclosure can have binding activity with the AM14 monoclonal antibody, and a wild-type RSV F protein before artificial mutation has no binding activity with the AM14 monoclonal antibody. A Synagis^{®} binding experiment shows that the mutant provided in the embodiments of the present disclosure and the wild-type RSV F protein before artificial mutation have strong binding activity with Synagis^{®}. The result indicates that the introduced mutation successfully stabilizes the RSV F protein in the prefusion trimer state, and the RSV F protein folds well. Accordingly, any combination of the technical solutions provided in the above embodiments of the present disclosure can solve the problems in the prior art and achieve same or similar technical effects.

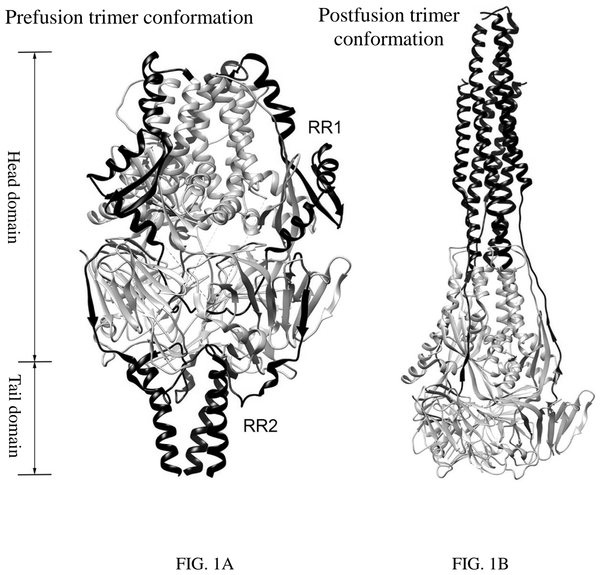

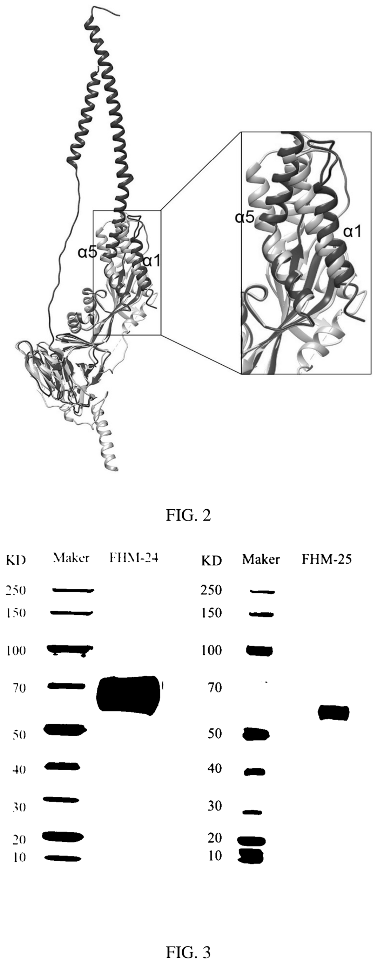

In the embodiments of the present disclosure, amino acids at positions 73 to 96 and amino acids at positions 216 to 240 are located in an α1 helix and an α5 helix (shown in FIG. 2) of a head domain (shown in FIG. 1A) of the prefusion wild-type RSV F protein trimer. The head domain is defined in order to describe a structure of the RSV F protein visually in the present disclosure. Amino acids at positions 1 to 485 are defined as the head domain. An amino acid at position 486 to a C-terminus of the F protein are defined as a tail domain. An approximate spatial positions of the head domain and the tail domain are shown in FIG. 1A. In the embodiments of the present disclosure, the α1 helix or the α5 helix refers to a first or fifth α helix from an N-terminal of the head domain, and is approximately located at the amino acids at positions 73 to 96 and the amino acids at positions 216 to 240 in a primary structure of the RSV F protein. Based on research results of the present disclosure, it may be considered that mutation of any amino acid in the α1 helix or the α5 helix of the head domain of the prefusion RSV F protein trimer stabilizes the RSV F protein in the prefusion trimer state. Thus, the amino acids at positions 73 to 96 and the amino acids at positions 216 to 240 described in the present disclosure may have one or more position shifts, for example, 2, 3, 4, 5, 6, 7, 8, 9, and 10 amino acid position shifts, which are also considered to be included in a scope of protection of the present disclosure.

In the embodiments of the present disclosure, position numbers of the amino acids are calculated from an amino acid at a first position of an amino acid sequence of the wild-type RSV F protein which is used as a reference. For example, the amino acid at position 73 is calculated from methionine, an amino acid at a first position, of a sequence MELPILKA of the wild-type RSV F protein.

In the embodiments of the present disclosure, the artificial mutation may generally refer to a substitution, a deletion, or an insertion of an amino acid residue in the wild-type RSV F protein. However, in some embodiments, the artificial mutation refers only to a substitution of an amino acid residue. The mutant described in the present disclosure is obtained by the artificial mutation described above.

In some other embodiments, compared with the wild-type RSV F protein before artificial mutation, the mutant has a plurality of amino acid mutation sites. The plurality of amino acid mutation sites include:
(a) at least two sties selected from the amino acids at positions 73 to 96; or
(b) at least two sties selected from the amino acids at positions 216 to 240; or
(c) at least one site selected from the amino acids at positions 73 to 96, and at least one site selected from the amino acids at positions 216 to 240.

Further, in some other embodiments, compared with the wild-type RSV F protein before artificial mutation, the mutant has the plurality of amino acid mutation sites. The plurality of amino acid mutation sites may include a combination of any one of the following (a), (b) or (c) with (d):
(a) at least two sties selected from the amino acids at positions 73 to 96;
(b) at least two sties selected from the amino acids at positions 216 to 240;
(c) at least one site selected from the amino acids at positions 73 to 96, and at least one site selected from the amino acids at positions 216 to 240; and
(d) at least one site selected from L141, S190, L193, L195, I199, or L373.

In some embodiments, the plurality may be, for example, 2, 3, 4, 5, 6, 7, 8, 9, 10, or more.

In some embodiments, the amino acid mutation site may include at least one site selected from the amino acid at position 79, the amino acid at position 82, the amino acid at position 92, the amino acid at position 93, the amino acid at position 96, the amino acid at position 141, the amino acid at position 190, the amino acid at position 193, the amino acid at position 195, the amino acid at position 199, the amino acid at position 220, the amino acid at position 224, the amino acid at position 227, the amino acid at position 230, the amino acid at position 234, the amino acid at position 238, or the amino acid at position 373.

Further, in some other embodiments, the amino acid mutation site may include the following:
(1) the amino acid at position 92; or
(2) the amino acid at position 96; or
(3) the amino acid at position 227; or
(4) the amino acid at position 230; or
(5) the amino acid at position 238; or
(6) the amino acid at position 227 and the amino acid at position 230; or
(7) the amino acid at position 82 and the amino acid at position 227; or
(8) the amino acid at position 89 and the amino acid at position 227; or
(9) the amino acid at position 92 and the amino acid at position 227; or
(10) the amino acid at position 92 and the amino acid at position 230; or
(11) the amino acid at position 96 and the amino acid at position 227; or
(12) the amino acid at position 96 and the amino acid at position 230; or
(13) the amino acid at position 96 and the amino acid at position 238; or
(14) the amino acid at position 93 and the amino acid at position 227; or
(15) the amino acid at position 89 and the amino acid at position 230; or
(16) the amino acid at position 82 and the amino acid at position 230; or
(17) the amino acid at position 93 and the amino acid at position 230; or
(18) the amino acid at position 227 and the amino acid at position 238; or
(19) the amino acid at position 230 and the amino acid at position 238; or
(20) the amino acid at position 227 and the amino acid at position 234; or
(21) the amino acid at position 82 and the amino acid at position 234; or
(22) the amino acid at position 193 and the amino acid at position 195; or
(23) the amino acid at position 190, the amino acid at position 193, and the amino acid at position 195; or
(24) the amino acid at position 193 and the amino acid at position 199; or
(25) the amino acid at position 141 and the amino acid at position 373; or
(26) the amino acid at position 82 and the amino acid at position 224; or
(27) the amino acid at position 92 and the amino acid at position 238; or
(28) the amino acid at position 79 and the amino acid at position 220; or
(29) an amino acid at position 97 and an amino acid at position 291; or
(30) the amino acid at position 93 and the amino acid at position 234; or
(31) the amino acid at position 92 and the amino acid at position 234; or
(32) the amino acid at position 193, the amino acid at position 227, and the amino acid at position 230; or
(33) the amino acid at position 190, the amino acid at position 193, and the amino acid at position 230; or
(34) the amino acid at position 193 and the amino acid at position 230; or
(35) the amino acid at position 190, the amino acid at position 193, the amino acid at position 227, and the amino acid at position 230; or
(36) the amino acid at position 93, the amino acid at position 230, and the amino acid at position 234; or
(37) the amino acid at position 93, the amino acid at position 193, the amino acid at position 230, and the amino acid at position 234; or
(38) the amino acid at position 93, the amino acid at position 190, the amino acid at position 193, and the amino acid at position 234; or
(39) the amino acid at position 93, the amino acid at position 227, the amino acid at position 193, and the amino acid at position 234; or
(40) the amino acid at position 93, the amino acid at position 234, and the amino acid at position 373; or
(41) the amino acid at position 230, the amino acid at position 238, and the amino acid at position 373; or
(42) the amino acid at position 93, the amino acid at position 141, and the amino acid at position 234; or
(43) the amino acid at position 93, the amino acid at position 141, the amino acid at position 230, and the amino acid at position 234; or
(44) the amino acid at position 141, the amino acid at position 230, and the amino acid at position 238; or
(45) the amino acid at position 227, the amino acid at position 230, and the amino acid at position 373; or
(46) the amino acid at position 93, the amino acid at position 227, and the amino acid at position 234; or
(47) the amino acid at position 93, the amino acid at position 227, the amino acid at position 230, and the amino acid at position 234; or
(48) the amino acid at position 93, the amino acid at position 227, the amino acid at position 234, and the amino acid at position 373; or
(49) the amino acid at position 227, the amino acid at position 230, the amino acid at position 238, and the amino acid at position 373.

Furthermore, in some embodiments, the amino acid mutation site may include the following:
(1) the amino acid at position 92; or
(2) the amino acid at position 96; or
(3) the amino acid at position 227; or
(4) the amino acid at position 230; or
(5) the amino acid at position 238; or
(6) the amino acid at position 227 and the amino acid at position 230; or
(7) the amino acid at position 92 and the amino acid at position 227; or
(8) the amino acid at position 92 and the amino acid at position 230; or
(9) the amino acid at position 227 and the amino acid at position 238; or
(10) the amino acid at position 230 and the amino acid at position 238; or
(11) the amino acid at position 93 and the amino acid at position 234; or
(12) the amino acid at position 92 and the amino acid at position 234; or
(13) the amino acid at position 193, the amino acid at position 227, and the amino acid at position 230; or
(14) the amino acid at position 190, the amino acid at position 193, the amino acid at position 227, and the amino acid at position 230; or
(15) the amino acid at position 93, the amino acid at position 230, and the amino acid at position 234; or
(16) the amino acid at position 93, the amino acid at position 234, and the amino acid at position 373; or
(17) the amino acid at position 230, the amino acid at position 238, and the amino acid at position 373; or
(18) the amino acid at position 93, the amino acid at position 141, and the amino acid at position 234; or
(19) the amino acid at position 93, the amino acid at position 141, the amino acid at position 230, and the amino acid at position 234; or
(20) the amino acid at position 141, the amino acid at position 230, and the amino acid at position 238; or
(21) the amino acid at position 227, the amino acid at position 230, and the amino acid at position 373; or
(22) the amino acid at position 93, the amino acid at position 227, and the amino acid at position 234; or
(23) the amino acid at position 93, the amino acid at position 227, the amino acid at position 234, and the amino acid at position 373; or
(24) the amino acid at position 190, the amino acid at position 193, and the amino acid at position 230; or
(25) the amino acid at position 193 and the amino acid at position 230; or
(26) the amino acid at position 93, the amino acid at position 193, the amino acid at position 230, and the amino acid at position 234; or
(27) the amino acid at position 93, the amino acid at position 190, the amino acid at position 193, and the amino acid at position 234; or
(28) the amino acid at position 93, the amino acid at position 227, the amino acid at position 193, and the amino acid at position 234; or
(29) the amino acid at position 93, the amino acid at position 227, the amino acid at position 230, and the amino acid at position 234; or
(30) the amino acid at position 227, the amino acid at position 230, the amino acid at position 238, and the amino acid at position 373.

To further improve the stability of the prefusion RSV F protein trimer, in some embodiments, the amino acid mutation site further includes at least one site selected from amino acids at positions 486 to 513.

A binding experiment of a prefusion trimer-specific monoclonal antibody AM14 shows that the mutant provided in embodiments of the present disclosure can have strong binding activity with the AM14 monoclonal antibody, and a wild-type RSV F protein before artificial mutation has no binding activity with the AM14 monoclonal antibody. A Synagis^{®} binding experiment shows that the mutant provided in the embodiments of the present disclosure and the wild-type RSV F protein before artificial mutation have strong binding activity with Synagis^{®}. The result indicates that the introduced mutation successfully stabilizes the RSV F protein in the prefusion trimer state, and the RSV F protein folds well. According to the embodiments of the present disclosure, an accelerated stability test is also performed on the expressed mutant of the F protein. A result shows that the mutant has no significant change in binding activity of an AM14 monoclonal antibody when the mutant is stored at 37°C for 4 weeks. It indicates that a prefusion mutant protein trimer remains stable. An animal immunological experiment is also performed in the embodiments of the present disclosure. A result shows that relative to the wild-type RSV F protein before artificial mutation, the mutant provided in the embodiments of the present disclosure can induce neutralizing antibodies with a higher titer. Accordingly, any combination of the technical solutions provided in the above embodiments of the present disclosure can solve the problems in the prior art and achieve same or similar technical effects.

In the embodiments of the present disclosure, the amino acids at positions 486 to 513 are located in the tail domain (shown in FIG. 1A) of the prefusion wild-type RSV F protein trimer. The tail domain is defined in order to describe the structure of the RSV F protein visually in the present disclosure. The amino acids at positions 1 to 485 are defined as the head domain. The amino acid at position 486 to the C-terminus of the F protein are defined as the tail domain. The approximate spatial positions of the head domain and the tail domain are shown in FIG. 1A. Based on research results of the present disclosure, it may be considered that mutation of any amino acid at positions 486 to 513 of the tail domain of the prefusion RSV F protein trimer further stabilizes a prefusion trimer structure of the RSV F protein. Thus, the amino acids at positions 486 to 513 described in the present disclosure may have one or more position shifts, for example, 2, 3, 4, 5, 6, 7, 8, 9, and 10 amino acid position shifts, which are also considered to be included in a scope of protection of the present disclosure.

Accordingly, in some embodiments, the amino acid mutation site further includes at least one site selected from the amino acid at position 501, the amino acid at position 502, the amino acid at position 505, the amino acid at position 509, or the amino acid at position 512.

Further, in some other embodiments, the amino acid mutation site may further include the following:
(1) the amino acid at position 509; or
(2) the amino acid at position 505; or
(3) the amino acid at position 502; or
(4) the amino acid at position 501; or
(5) the amino acid at position 505 and the amino acid at position 509; or
(6) the amino acid at position 502 and the amino acid at position 509; or
(7) the amino acid at position 501 and the amino acid at position 509; or
(8) the amino acid at position 502 and the amino acid at position 505; or
(9) the amino acid at position 501 and the amino acid at position 505; or
(10) the amino acid at position 502, the amino acid at position 505, and the amino acid at position 509; or
(11) the amino acid at position 501, the amino acid at position 505, and the amino acid at position 509.

Furthermore, in some embodiments, the amino acid mutation site further includes the following:
(1) the amino acid at position 509; or
(2) the amino acid at position 505; or
(3) the amino acid at position 505 and the amino acid at position 509; or
(4) the amino acid at position 502 and the amino acid at position 509; or
(5) the amino acid at position 501 and the amino acid at position 509.

The RSV F protein has two subtypes, that is, subtype A and subtype B. The two subtypes have high sequence conservation, and their amino acid sequence homologies reach 90%. Amino acid sequence homologies between different strains in the subtype A and the subtype B are higher. An RSV F protein of each subtype and each strain consists of 574 amino acids. In view of high conservation of amino acid sequences between the different subtypes of RSV F proteins, the mutation solutions provide in the present disclosure are applicable to any strain sequence of the RSV F protein subtype A and subtype B or a strain sequence that may appear in the future. A sequence of the wild-type RSV F protein in the embodiments of the present disclosure may be derived from any known or future likely F protein sequence, and includes, but is not limited to, an F protein sequence of any RSV strain subtype A or subtype B, such as an F protein sequence of strain A2 of subtype A shown in SEQ ID NO. 1-2, an F protein sequence of strain ON1 of subtype A shown in SEQ ID NO. 3 , and an F protein sequence of a strain of subtype B shown in SEQ ID NO. 4-6. Since sequences of wild-type RSV F proteins between RSV subtypes and strains have high conservation or identity, it is permissible for the sequences of the wild-type RSV F proteins to contain a minor difference. The minor difference may be a substitution, a deletion or an insertion between amino acid residues. Thus, in some embodiments, the sequences of the wild-type RSV F proteins may have identity greater than 90%, for example, greater than 91%, greater than 92%, greater than 93%, greater than 94%, greater than 95%, greater than 96%, greater than 97%, greater than 98%, and greater than 99%, and have same or substantially same biological functions.

Based on the research results of the present disclosure, in some embodiments, the wild-type RSV F protein may be in the following form:
(a) an F0 precursor protein comprising two protease cleavage sites; or
(b) an F0 precursor protein that is incapable of binding to a protease; or
(c) a combination of an independent F1 polypeptide and an independent F2 polypeptide; or
(d) a combination of an F2 polypeptide, a linker, and an F1 polypeptide; or
(e) a combination of an F2' polypeptide, a linker, and an F1' polypeptide, where the F2' polypeptide is 1 to 10 amino acid residues less than the F2 polypeptide in an N-terminal direction, and the F1' polypeptide is 1 to 10 amino acid residues less than the F1 polypeptide in a C-terminal direction.

In some embodiments, the wild-type RSV F protein may be an original F0 precursor protein, that is, a protein including an F1 polypeptide, an F2 polypeptide, a P27 peptide, and optionally including a transmembrane domain and an intracellular domain. The F0 precursor protein includes a cleavage site for a protease. The protease may be, for example, furin, trypsin, factor Xa, thrombin, or cathepsin L. Typically, the protease is furin. During its expression, the F0 precursor protein is naturally cleaved by the protease in an expression system, thus losing the P27 peptide. The F1 polypeptide and the F2 polypeptide together form the F protein in a mature form. In some other embodiments, the wild-type RSV F protein may be an F0 precursor protein that is unable to bind a protease, that is, a protein including an F1 polypeptide, an F2 polypeptide, a P27 peptide, optionally including a transmembrane domain and an intracellular domain, but unable to bind a protease. Thus the P27 peptide is retained. The protease may be, for example, furin, trypsin, factor Xa, thrombin, or cathepsin L. Typically, the protease is furin. A reason for an inability to bind to a protease may be a deletion, a substitution or an insertion of amino acid residues at a cleavage site which is used for binding a protease. In some other embodiments, the wild-type RSV F protein may be a combination of an independent F1 polypeptide and an independent F2 polypeptide, optionally a protein including a transmembrane domain and an intracellular domain. The F1 polypeptide and the F2 polypeptide spontaneously form an F2-F1 polypeptide heterodimer. In vitro expression, the F1 polypeptide and the F2 polypeptide need to be expressed separately. In some other embodiments, the wild-type RSV F protein may be a combination of an F2 polypeptide, a linker and an F1 polypeptide, that is, a protein including an F1 polypeptide, an F2 polypeptide, and a linker (Linker-1), optionally including a transmembrane domain and an intracellular domain. In some other embodiments, the wild-type RSV F protein may be a combination of an F2' polypeptide, a linker, and an F1' polypeptide. The F2' polypeptide is 1 to 10 amino acid residues less than the F2 polypeptide in an N-terminal direction. The F1' polypeptide is 1 to 10 amino acid residues less than the F1 polypeptide in a C-terminal direction. That is, a longer sequence including the P27 peptide in a sequence of the F0 is replaced with a linker. Some studies show that replacing the P27 peptide with a short linker can increase protein expression and a trimer content (Ref: Krarup A. et al. Nat. Commun., 2015, 6:8143). Thus, in one embodiment, a sequence at positions 107 to 138 (RARRELPRFMNYTLNNAKKTNVTLSKKRKRRF) including the P27 peptide and a furin cleavage site is replaced with a QARGSGSGRS sequence.

In some embodiments, the wild-type RSV F protein before artificial mutation may include sequences shown in SEQ ID No. 7-34 in a sequence table, or include sequences having identity greater than 90% , for example, greater than 91%, greater than 92%, greater than 93%, greater than 94%, greater than 95%, greater than 96%, greater than 97%, greater than 98%, and greater than 99%, and having same or substantially same biological functions as the sequences shown in SEQ ID No. 7-34.

Based on the research results of the present disclosure, in some embodiments, amino acid residue combinations in which the amino acid mutation sites are substituted are also provided. The amino acid mutation site includes at least one of the following:
(1) the amino acid at position 79 is substituted by C, and the amino acid at position 220 is substituted by C; or
(2) the amino acid at position 82 is substituted by C, and the amino acid at position 224 is substituted by C; or
(3) the amino acid at position 92 is substituted by C, and the amino acid at position 234 is substituted by C; or
(4) the amino acid at position 93 is substituted by C, and the amino acid at position 234 is substituted by C; or
(5) the amino acid at position 82 is substituted by L; or
(6) the amino acid at position 92 is substituted by L, I, M, F, Y, or W; or
(7) the amino acid at position 96 is substituted by M or Y; or
(8) the amino acid at position 141 is substituted by M, F, Y, or W; or
(9) the amino acid at position 190 is substituted by V; or
(10) the amino acid at position 193 is substituted by M, F, Y, or W; or
(11) the amino acid at position 195 is substituted by M, F, Y, or W; or
(12) the amino acid at position 199 is substituted by M, F, Y, or W; or
(13) the amino acid at position 227 is substituted by A, V, L, I, M, S, T, or Q; or
(14) the amino acid at position 230 is substituted by F; or
(15) the amino acid at position 238 is substituted by L, I, M, F, Y, or W; or
(16) the amino acid at position 373 is substituted by M, F, or Y.

Further, in some embodiments, the amino acid mutation site includes the following:
(1) the amino acid at position 92 is substituted by I, M, F, Y, or W; or
(2) the amino acid at position 96 is substituted by Y; or
(3) the amino acid at position 227 is substituted by M, V, or I; or
(4) the amino acid at position 230 is substituted by F; or
(5) the amino acid at position 238 is substituted by L, I, M, or W; or
(6) the amino acid at position 227 is substituted by M or V, and the amino acid at position 230 is substituted by F; or
(7) the amino acid at position 82 is substituted by L, and the amino acid at position 227 is substituted by M; or
(8) the amino acid at position 92 is substituted by M or L, and the amino acid at position 227 is substituted by M or V; or
(9) the amino acid at position 92 is substituted by M or L, and the amino acid at position 230 is substituted by F; or
(10) the amino acid at position 96 is substituted by Y, and the amino acid at position 227 is substituted by M or V; or
(11) the amino acid at position 96 is substituted by Y, and the amino acid at position 230 is substituted by F; or
(12) the amino acid at position 96 is substituted by Y, and the amino acid at position 238 is substituted by L, I, M, or W; or
(13) the amino acid at position 89 is substituted by L, and the amino acid at position 230 is substituted by F; or
(14) the amino acid at position 227 is substituted by M, and the amino acid at position 238 is substituted by L, I, M, or W; or
(15) the amino acid at position 230 is substituted by F, and the amino acid at position 238 is substituted by L, I, M, or W; or
(16) the amino acid at position 193 is substituted by M, and the amino acid at position 195 is substituted by M, F, or Y; or
(17) the amino acid at position 190 is substituted by V, the amino acid at position 193 is substituted by M, and the amino acid at position 195 is substituted by M, F, or Y; or
(18) the amino acid at position 193 is substituted by M, and the amino acid at position 199 is substituted by M or F; or
(19) the amino acid at position 141 is substituted by M or F, and the amino acid at position 373 is substituted by M, F, or Y; or
(20) the amino acid at position 82 is substituted by C, and the amino acid at position 224 is substituted by C; or
(21) the amino acid at position 92 is substituted by C, and the amino acid at position 238 is substituted by C; or
(22) the amino acid at position 79 is substituted by C, and the amino acid at position 220 is substituted by C; or
(23) the amino acid at position 93 is substituted by C, and the amino acid at position 234 is substituted by C; or
(24) the amino acid at position 92 is substituted by C, and the amino acid at position 234 is substituted by C; or
(25) the amino acid at position 193 is substituted by M, the amino acid at position 227 is substituted by M or V, and the amino acid at position 230 is substituted by F; or
(26) the amino acid at position 190 is substituted by V, the amino acid at position 193 is substituted by M, and the amino acid at position 230 is substituted by F; or
(27) the amino acid at position 193 is substituted by M, and the amino acid at position 230 is substituted by F; or
(28) the amino acid at position 190 is substituted by V, the amino acid at position 193 is substituted by M, the amino acid at position 227 is substituted by M or V, and the amino acid at position 230 is substituted by F; or
(29) the amino acid at position 93 is substituted by C, the amino acid at position 230 is substituted by F, and the amino acid at position 234 is substituted by C; or
(30) the amino acid at position 93 is substituted by C, the amino acid at position 193 is substituted by M, the amino acid at position 230 is substituted by F, and the amino acid at position 234 is substituted by C; or
(31) the amino acid at position 93 is substituted by C, the amino acid at position 190 is substituted by V, the amino acid at position 193 is substituted by M, and the amino acid at position 234 is substituted by C; or
(32) the amino acid at position 93 is substituted by C, the amino acid at position 227 is substituted by M or V, and the amino acid at position 234 is substituted by C; or
(33) the amino acid at position 93 is substituted by C, the amino acid at position 193 is substituted by M, the amino acid at position 227 is substituted by M or V, and the amino acid at position 234 is substituted by C; or
(34) the amino acid at position 93 is substituted by C, the amino acid at position 234 is substituted by C, and the amino acid at position 373 is substituted by M, F, or Y; or
(35) the amino acid at position 230 is substituted by F, the amino acid at position 238 is substituted by L, I, M, or W, and the amino acid at position 373 is substituted by M, F, or Y; or
(36) the amino acid at position 93 is substituted by C, the amino acid at position 141 is substituted by M or F, and the amino acid at position 234 is substituted by C; or
(37) the amino acid at position 93 is substituted by C, the amino acid at position 141 is substituted by M or F, the amino acid at position 230 is substituted by F, and the amino acid at position 234 is substituted by C; or
(38) the amino acid at position 141 is substituted by M or F, the amino acid at position 230 is substituted by F, and the amino acid at position 238 is substituted by L, I, M, or W; or
(39) the amino acid at position 227 is substituted by M or V, the amino acid at position 230 is substituted by F, and the amino acid at position 373 is substituted by M, F, or Y; or
(40) the amino acid at position 93 is substituted by C, the amino acid at position 227 is substituted by M or V, the amino acid at position 230 is substituted by F, and the amino acid at position 234 is substituted by C; or
(41) the amino acid at position 93 is substituted by C, the amino acid at position 227 is substituted by M or V, the amino acid at position 234 is substituted by C, and the amino acid at position 373 is substituted by M, F or Y; or
(42) the amino acid at position 227 is substituted by M or V, the amino acid at position 230 is substituted by F, the amino acid at position 238 is substituted by L, I, M, or W, and the amino acid at position 373 is substituted by M, F or Y.

Furthermore, in some embodiments, the amino acid mutation site may include the following:
(1) the amino acid at position 92 is substituted by I, M, F, Y, or W; or
(2) the amino acid at position 96 is substituted by Y; or
(3) the amino acid at position 227 is substituted by M or V; or
(4) the amino acid at position 230 is substituted by F; or
(5) the amino acid at position 238 is substituted by L, I, M, or W; or
(6) the amino acid at position 227 is substituted by M or V, and the amino acid at position 230 is substituted by F; or
(7) the amino acid at position 92 is substituted by M, and the amino acid at position 227 is substituted by M or V; or
(8) the amino acid at position 92 is substituted by M, and the amino acid at position 230 is substituted by F; or
(9) the amino acid at position 227 is substituted by M or V, and the amino acid at position 238 is substituted by L or I; or
(10) the amino acid at position 230 is substituted by F, and the amino acid at position 238 is substituted by L or I; or
(11) the amino acid at position 96 is substituted by Y, and the amino acid at position 227 is substituted by M or V; or
(12) the amino acid at position 96 is substituted by Y, and the amino acid at position 230 is substituted by F; or
(13) the amino acid at position 96 is substituted by Y, and the amino acid at position 238 is substituted by L, I, M, or W; or
(14) the amino acid at position 93 is substituted by C, and the amino acid at position 234 is substituted by C; or
(15) the amino acid at position 92 is substituted by C, and the amino acid at position 234 is substituted by C; or
(16) the amino acid at position 193 is substituted by M, the amino acid at position 227 is substituted by M or V, and the amino acid at position 230 is substituted by F; or
(17) the amino acid at position 190 is substituted by V, the amino acid at position 193 is substituted by M, the amino acid at position 227 is substituted by M or V, and the amino acid at position 230 is substituted by F; or
(18) the amino acid at position 93 is substituted by C, the amino acid at position 230 is substituted by F, and the amino acid at position 234 is substituted by C; or
(19) the amino acid at position 93 is substituted by C, the amino acid at position 234 is substituted by C, and the amino acid at position 373 is substituted by M or F; or
(20) the amino acid at position 230 is substituted by F, the amino acid at position 238 is substituted by L or I, and the amino acid at position 373 is substituted by M or F; or
(21) the amino acid at position 93 is substituted by C, the amino acid at position 141 is substituted by M or F, and the amino acid at position 234 is substituted by C; or
(22) the amino acid at position 93 is substituted by C, the amino acid at position 141 is substituted by M or F, the amino acid at position 230 is substituted by F, and the amino acid at position 234 is substituted by C; or
(23) the amino acid at position 141 is substituted by M or F, the amino acid at position 230 is substituted by F, and the amino acid at position 238 is substituted by L or I; or
(24) the amino acid at position 227 is substituted by M or V, the amino acid at position 230 is substituted by F, and the amino acid at position 373 is substituted by M or F; or
(25) the amino acid at position 93 is substituted by C, the amino acid at position 227 is substituted by M or V, and the amino acid at position 234 is substituted by C; or
(26) the amino acid at position 93 is substituted by C, the amino acid at position 227 is substituted by M or V, the amino acid at position 234 is substituted by C, and the amino acid at position 373 is substituted by M or F; or
(27) the amino acid at position 190 is substituted by V, the amino acid at position 193 is substituted by M, and the amino acid at position 230 is substituted by F; or
(28) the amino acid at position 193 is substituted by M, and the amino acid at position 230 is substituted by F; or
(29) the amino acid at position 93 is substituted by C, the amino acid at position 193 is substituted by M, the amino acid at position 230 is substituted by F, and the amino acid at position 234 is substituted by C; or
(30) the amino acid at position 93 is substituted by C, the amino acid at position 190 is substituted by V, the amino acid at position 193 is substituted by M, and the amino acid at position 234 is substituted by C; or
(31) the amino acid at position 93 is substituted by C, the amino acid at position 227 is substituted by M or V, the amino acid at position 193 is substituted by M, and the amino acid at position 234 is substituted by C; or
(32) the amino acid at position 93 is substituted by C, the amino acid at position 227 is substituted by M or V, the amino acid at position 230 is substituted by F, and the amino acid at position 234 is substituted by C; or
(33) the amino acid at position 227 is substituted by M or V, the amino acid at position 230 is substituted by F, the amino acid at position 238 is substituted by L or I, and the amino acid at position 373 is substituted by M or F.

To further improve the stability of the prefusion RSV F protein trimer, in some embodiments, an amino acid mutation site of the tail domain is also introduced. The amino acid mutation site includes the following:
(1) the amino acid at position 501 is substituted by V, L, I, M, F, W, Y, P, or R; or
(2) the amino acid at position 502 is substituted by V, L, I, M, F, W, Y, P, or R; or
(3) the amino acid at position 505 is substituted by M or W; or
(4) the amino acid at position 509 is substituted by G, A, V, L, I, M, F, W, P, S, T, C, Y, Q, K, R, or H; or
(5) the amino acid at position 512 is substituted by I, F, M, Y, R, or K.

Further, in some embodiments, the amino acid mutation site further includes the following:
(1) the amino acid at position 509 is substituted by F, M, or L; or
(2) the amino acid at position 505 is substituted by W; or
(3) the amino acid at position 502 is substituted by Y; or
(4) the amino acid at position 501 is substituted by M; or
(5) the amino acid at position 505 is substituted by W, and the amino acid at position 509 is substituted by F, L, or M; or
(6) the amino acid at position 502 is substituted by Y, and the amino acid at position 509 is substituted by F; or
(7) the amino acid at position 501 is substituted by M, and the amino acid at position 509 is substituted by F; or
(8) the amino acid at position 502 is substituted by Y, and the amino acid at position 505 is substituted by W; or
(9) the amino acid at position 501 is substituted by M, and the amino acid at position 505 is substituted by W; or
(10) the amino acid at position 502 is substituted by Y, the amino acid at position 505 is substituted by W, and the amino acid at position 509 is substituted by F; or
(11) the amino acid at position 501 is substituted by M, the amino acid at position 505 is substituted by W, and the amino acid at position 509 is substituted by F.

Furthermore, in some embodiments, the amino acid mutation site further includes the following:
(1) the amino acid at position 509 is substituted by F, M, or L; or
(2) the amino acid at position 505 is substituted by W; or
(3) the amino acid at position 505 is substituted by W, and the amino acid at position 509 is substituted by F or L; or
(4) the amino acid at position 502 is substituted by Y, and the amino acid at position 509 is substituted by F; or
(5) the amino acid at position 501 is substituted by M, and the amino acid at position 509 is substituted by F.

In some embodiments, specific instances of the mutant may include sequences shown in SEQ ID No. 35-166 in the sequence table, or include sequences having identity greater than 90% , for example, greater than 91%, greater than 92%, greater than 93%, greater than 94%, greater than 95%, greater than 96%, greater than 97%, greater than 98%, and greater than 99%, and having same or substantially same biological functions as the sequences shown in SEQ ID No. 7-34.

A C-terminal of the full-length RSV F protein includes one transmembrane region and one intracellular region. In some embodiments, the transmembrane region and/or the intracellular region may be retained or removed. To facilitate secretory expression, in some embodiment, the transmembrane region and the intracellular region of the C-terminus of the F protein are removed to obtain a soluble F protein. In some other embodiments of the present disclosure, the intracellular region and the entire transmembrane region or a portion of the transmembrane region may be removed, or a longer sequence including the transmembrane region and the intracellular region at the C-terminus may be removed. In some specific examples of the present disclosure, amino acids at positions 514 to 574 are removed from the C-terminus.

In some embodiment, the mutant may be in a trimeric form. A natural structure of the RSV F protein is a homotrimer. Thus, preferably, in some embodiment, the trimeric form may be a homotrimer form. With the transmembrane region and the intracellular region retained or removed, the C-terminus of the F protein may be directly or indirectly linked to an exogenous trimerization domain to facilitate formation of a trimeric structure. An exogenous multimerization domain is known in the art, such as a GCN4 leucine zipper (Ref: Sliepen K. et al. J. Biol. Chem., 2015, 290:7436-7442.), collagen (Ref: McAlinden A. et al. J. Biol. Chem., 2003, 278:42200-42207), and T4 foldon (Ref: 1. McLellan J. S. et al. Science, 2013, 342:592-598. 2. Joyce M. G. et al. Nat. Struct. Mol. Biol., 2016, 23:811-820). In some specific instances, the C-terminus of the F protein is linked to the T4 foldon to facilitate trimerization of the F protein.

In some embodiments, the mutant may be modified in order to implement some functions without influencing biological activity, for example, immunogenicity, of the mutant as a whole. The modification may be at least one of glycosylation modification, phosphorylation modification, methylation modification, coenzyme modification, or acylation modification.

When the exogenous multimerization domain is linked to the F protein, the exogenous multimerization domain may be directly linked to the C-terminus of the F protein, or may be linked via a linker (Linker-2). The linker may be long or short. In some embodiments, the linker may be, for example, a GS repeat sequence, a GGS repeat sequence, an SAIG repeat sequence, a GSS repeat sequence, or a GG repeat sequence. These linkers, as well as other similar linkers, are well known in the art and can be used in the mutant of the present disclosure. In some specific instances, the F protein is linked to the exogenous multimerization domain via an SAIG linker.

In order to implement some specific purposes or functions, in some embodiments, the mutant may further includes a functional tag. The functional tag may be added in an appropriate place. For example, the functional tag may be one or more of a signal peptide, a tag, or an immune enhancing peptide. The signal peptide may be used to facilitate protein expression. The tag may be a Flag tag, an enhanced green fluorescent protein (eGFP), a flutathione S-transferase (GST), etc., and may be used for detection, purification, isolation, etc. The above functional sequences may be used in any combination. In some embodiments, in order to facilitate purification of a target protein, the constructed mutant of the F protein may contain a His-tag (formed by linking a plurality of His-tags), a streptomycin tag II, and an AviTag, and may also contain a tobacco etch virus (TEV) protein cleavage site for removal of an additional sequence. The additional sequence is well known to those skilled in the art. Addition or absence of the additional sequence does not influence the biological functions of the F protein. In some other examples, the F protein is linked to the exogenous multimerization domain and then linked to the His-tag (HHHHHHHH) via a GSGSGS linker to facilitate the purification of the target protein. In some other examples, the streptomycin tag II, the AviTag , and the TEV cleavage site are also linked besides the His-tag. A sequence of the mutant disclosed in the present disclosure does not include these additional tag sequences.

In the present disclosure, the mutant of the wild-type RSV F protein may be prepared according to contents of the present disclosure by conventional means in the prior art, such as loading a nucleotide sequence encoding the mutant into a suitable vector for expression and purification in a corresponding expression system. Several novel expression vectors for a respiratory syncytial virus are disclosed in China Patent Publication No. CN111166881A, CN106986942A and CN106986943A by Li Qiming, etc. The mutant of the RSV F protein may be expressed by using suitable host cells known in the art, for example, E. coli, yeast cells, insect cells, or mammalian cells. Instances of an E. coli expression system include, for example, GI698, ER2566, BL21 (DE3), XA90, DH(5a), B834 (DE3), and BLR (DE3). Instances of a yeast expression systems include, for example, Saccharomyces cerevisiae, Pichia pastoris, and Hansenula. Instances of the insect cells include, for example, Sf9 cells, Sf21 cells, Tn5 cells, Schneider S2 cells, and High Five cells. Instances of the mammalian cells include, for example, CHO cells, human embryonic kidney cells, NIH-3T3 cells, 293-T cells, Vero cells, and HeLa cells. An obtained protein may be purified through any suitable method, for example, salting out, precipitation, dialysis or ultrafiltration, molecular sieve chromatography, ion exchange chromatography, hydrophobic chromatography, and affinity chromatography.

In another aspect of the present disclosure, virus-like particles (VLPs) are provided. The virus-like particles includes the mutant of the present disclosure. The virus-like particles may be prepared through any suitable method in the prior art. For example, the VLPs are expressed by using specific insect or mammalian cell expression systems.

In another aspect of the present disclosure, an isolated nucleic acid molecule is provided. The isolated nucleic acid molecule encodes the mutant of the present disclosure. In some embodiments, the isolated nucleic acid molecule is a deoxyribonucleic acid (DNA) molecule. Generally, codons encoding amino acids of the mutant described in the embodiments of the present disclosure can be optimized to optimize their expression in a host cell. In some other embodiments, the isolated nucleic acid molecule is a ribonucleic acid (RNA) molecule. The RNA molecule includes a messenger RNA (mRNA) molecule.

In another aspect of the present disclosure, a vector is provided. The vector includes a sequence of the nucleic acid molecule of the present disclosure or the mutant of the present disclosure. In some embodiments, the vector is a protein expression vector. In some other embodiments, the vector is a gene delivery vector, such as a viral vector or a non-viral vector. In some other embodiments, the viral vector includes a retrovirus vector, a lentivirus vector, an adenovirus vector, an adeno-associated virus vector, a poxvirus vector, a herpes simplex viral vector or a Sendai viral vector. The vector may include regulatory sequences such as promoters and terminators, as well as tag sequences such as drug resistance genes and reporter genes. Moreover, the vector may also include a sequence encoding a suicide gene. In some other embodiments, the vector is a non-viral vector. In some other embodiment, the non-viral vector includes a liposome, a cationic polymer vector, a lipid nanoparticle (LNP) vector or a multifunctional envelope-type nano vector.

In another aspect of the present disclosure, a host cell is provided. The host cell includes the nucleic acid molecule described in the present disclosure or the vector described in the present disclosure.

In another aspect of the present disclosure, a protein conjugate is provided. The protein conjugate includes the mutant described in the present disclosure.

In another aspect of the present disclosure, a pharmaceutical composition is provided. The pharmaceutical composition includes the mutant of the present disclosure, the isolated nucleic acid molecule of the present disclosure, the vector of the present disclosure, the host cell of the present disclosure or the protein conjugate of the present disclosure, and a pharmaceutically acceptable carrier. The pharmaceutically acceptable carrier may be any pharmaceutically acceptable additive, for example, physiological saline, a cell culture medium, glucose, water for injection, glycerol, amino acids and their combinations, a stabilizer, a surfactant, a preservative, or an isotonic agent. In some embodiments, the pharmaceutical composition may be an immunogenic composition. In some embodiments, the pharmaceutical composition is a vaccine. The vaccine includes the mutant of the present disclosure, the isolated nucleic acid molecule of the present disclosure, the vector of the present disclosure, the host cell of the present disclosure or the protein conjugate of the present disclosure. The vaccine is a prophylactic or therapeutic vaccine. In some embodiments, the pharmaceutical composition may include one or more different mutants of the present disclosure, isolated nucleic acid molecules of the present disclosure, vectors of the present disclosure, host cells of the present disclosure, or protein conjugates of the present disclosure, for example, two or three of them. In some embodiments, the vaccine is a protein vaccine, a nucleic acid vaccine, or a vector vaccine.

For the protein vaccine, in some embodiments, the vaccine may further include an immunomodulator, such as an adjuvant. Any suitable adjuvant may be selected, for example, aluminum hydroxide, aluminum phosphate, MF59, or CpG. In some embodiments, the immunomodulator further includes an immunopotentiator, for example, a cytokine, a chemokine pathogen-associated molecular pattern (PAMP), a TLR-ligand, an immunostimulatory sequence, a DNA containing Cytosine-phosphate-Guanine oligodeoxynucleotides (CpG), a double-stranded RNA (dsRNA), a ligand of an endocytotic pattern recognition receptor, lipopolysaccharide (LPS), quillaja saponin, and Tucaresol.

In another aspect of the present disclosure, use of the mutant of the present disclosure, the isolated nucleic acid molecule of the present disclosure, the vector of the present disclosure, the host cell of the present disclosure, the protein conjugate of the present disclosure, or the pharmaceutical composition of the present disclosure for preparation of a product for preventing or treating an RSV infection is provided.

In some embodiments, a method for eliciting an immune response to the RSV in a subject is also provided. The method includes administering to a subject an effective dose of the mutant of the present disclosure, the isolated nucleic acid molecule of the present disclosure, the vector of the present disclosure, the host cell of the present disclosure, the protein conjugate of the present disclosure, or the pharmaceutical composition of the present disclosure. In some embodiments, the immune response may produce one or more neutralizing antibodies in the subject. In some embodiments, the immune response may elicit cellular immunity in the subject. In some embodiments, the immune response is a reduction or inhibition of RSV replication in the subject. In some embodiments, the administration may be one or more times of administration. In some embodiments, an administration method may be intramuscular injection, intraperitoneal injection, subcutaneous injection, mucosal immunization, etc.

The mutant of the present disclosure, the isolated nucleic acid molecule of the present disclosure, the vector of the present disclosure, the host cell of the present disclosure, the protein conjugate of the present disclosure, or the pharmaceutical composition of the present disclosure may be administered with other drugs including immunogenic compositions simultaneously or jointly.

In another aspect of the present disclosure, use of the mutant of the present disclosure, the isolated nucleic acid molecule of the present disclosure, the vector of the present disclosure, the host cell of the present disclosure or the protein conjugate of the present disclosure for preparation of an antibody against a prefusion conformation of the RSV F protein is provided.

In another aspect of the present disclosure, use of the mutant of the present disclosure, the isolated nucleic acid molecule of the present disclosure, the vector of the present disclosure, the host cell of the present disclosure or the protein conjugate of the present disclosure for detecting presence of RSV antibodies in a sample is provided. For example, whether a subject has the RSV infection is diagnosed by detecting the presence or absence of the RSV antibodies in a subject-derived sample.

In another aspect of the present disclosure, a method for preventing or treating an RSV infection is provided. An effective dose of the pharmaceutical composition of the present disclosure is administered to a patient.

The present disclosure has the beneficial effects as follows:
In the embodiments of the present disclosure, based on the structure analysis of the prefusion conformation and the postfusion conformation of the RSV F protein, other conformational change regions coupled with a conformational change of RR1 of the F protein from the prefusion conformation to the postfusion conformation are obtained. Stability mutation is introduced to prevent conformational movement, and/or mutation is directly introduced into interaction regions involved in RR1 to prevent a conformational transition, such that the F protein is stabilized to the prefusion trimer state. Moreover, the stability mutation is introduced into the tail domain of the F protein, and then the stability of the prefusion RSV F protein trimer structure is further enhanced. A binding experiment of a prefusion trimer-specific monoclonal antibody AM14 shows that the mutant provided in embodiments of the present disclosure can have strong binding activity with the AM14 monoclonal antibody, and a wild-type RSV F protein before artificial mutation has no binding activity with the AM14 monoclonal antibody. A Synagis^{®} binding experiment shows that the mutant provided in the embodiments of the present disclosure and the wild-type RSV F protein before artificial mutation have strong binding activity with Synagis^{®}. The result indicates that the introduced mutation successfully stabilizes the RSV F protein in the prefusion trimer state, and the RSV F protein folds well.

According to the embodiments of the present disclosure, an accelerated stability test is also performed on the expressed mutant of the F protein. A result shows that the mutant has no significant change in binding activity of an AM14 monoclonal antibody when the mutant is stored at 37°C for 4 weeks. It indicates that a prefusion mutant protein trimer remains stable.

An animal immunological experiment is also performed in the embodiments of the present disclosure. A result shows that relative to the wild-type RSV F protein before artificial mutation, the mutant provided in the present disclosure can induce neutralizing antibodies with a higher titer.

### BRIEF DESCRIPTION OF DRAWINGS

FIG. 1A is a structural diagram of a prefusion RSV F protein trimer, and FIG. 1B is a structural diagram of a postfusion RSV F protein trimer, where a head domain and a tail domain defined for visual description in the present disclosure are marked in the figures, refolding region 1 (RR1, located in the head domain) and refolding region 2 (RR2, located in a C-terminal of the head domain and the tail domain) that undergo large conformational changes during a transition of the F protein from a prefusion conformation to a postfusion conformation are marked in the figures, and FIG. 1A and FIG. 1B are drawn based on coordinate files with protein data bank (PDB) identifiers 4jhw and 3rrr respectively;
FIG. 2 is a structural superposition diagram of a prefusion conformation (lighter color) and a postfusion conformation (darker color) of an RSV F protein monomer, where during a transition of the F protein from the prefusion conformation to the postfusion conformation, RR1 of a head domain undergoes a large conformational change, and α1 and α5 correspondingly undergo obvious conformational movement; and an enlarged diagram on the right shows a conformational change before and after fusion of α1 and α5, the prefusion conformation and the postfusion conformation of the F protein monomer are drawn according to coordinate files with PDB identifiers 4jhw and 3rrr respectively;
FIG. 3 shows sodium dodecyl sulfate polyacrylamide gel electrophoresis (SDS-PAGE) diagrams of illustrative mutants FHM-24 and FHM-25 with mutation introduced into head domains according to an example of the present disclosure;

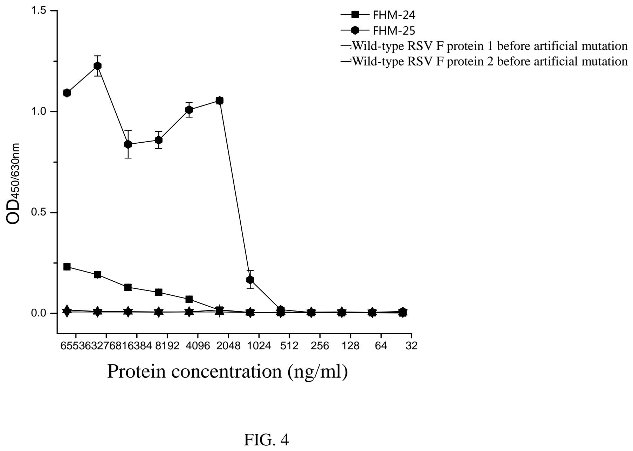

FIG. 4 is a graph showing results of AM14 monoclonal antibody binding experiments of illustrative mutants FHM-24 and FHM-25 with mutation introduced into head domains, a wild-type RSV F protein 1 before artificial mutation, and a wild-type RSV F protein 2 before artificial mutation according to an example of the present disclosure;

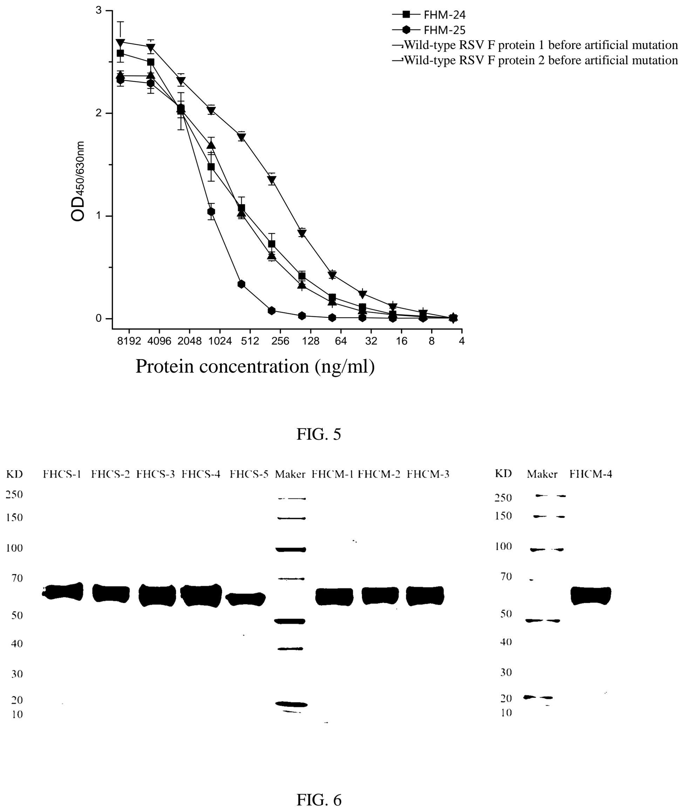

FIG. 5 is a graph showing results of Synagis^{®} binding experiments of illustrative mutants FHM-24 and FHM-25 with mutation introduced into head domains, a wild-type RSV F protein 1 before artificial mutation, and a wild-type RSV F protein 2 before artificial mutation according to an example of the present disclosure;
FIG. 6 shows SDS-PAGE diagrams of mutants FHCS-1 , FHCS-2, FHCS-3, FHCS-4, FHCM-5, FHCM-1, FHCM-2, FHCM-3 and FHCM-4, with purified proteins, constructed in an example of the present disclosure;

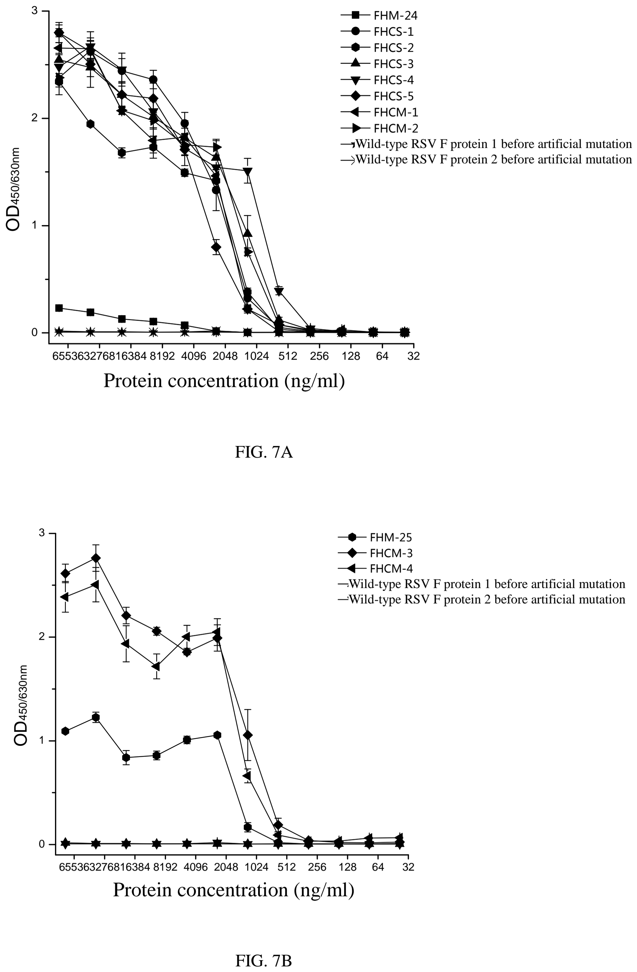

FIG. 7A is a comparison diagram of results of AM14 monoclonal antibody binding experiments of mutants FHCS-1, FHCS-2, FHCS-3, FHCS-4, FHCS-5, FHCM-1 and FHCM-2 constructed in an example of the present disclosure , a mutant FHM-24, wild-type RSV F protein 1 before artificial mutation, and a wild-type RSV F protein 2 before artificial mutation; and FIG. 7B is a comparison diagram of results of AM14 monoclonal antibody binding experiments of mutants FHCM-3 and FHCM-4 constructed in an example of the present disclosure, a mutant FHM-25, a wild-type RSV F protein 1 before artificial mutation, and a wild-type RSV F protein 2 before artificial mutation;

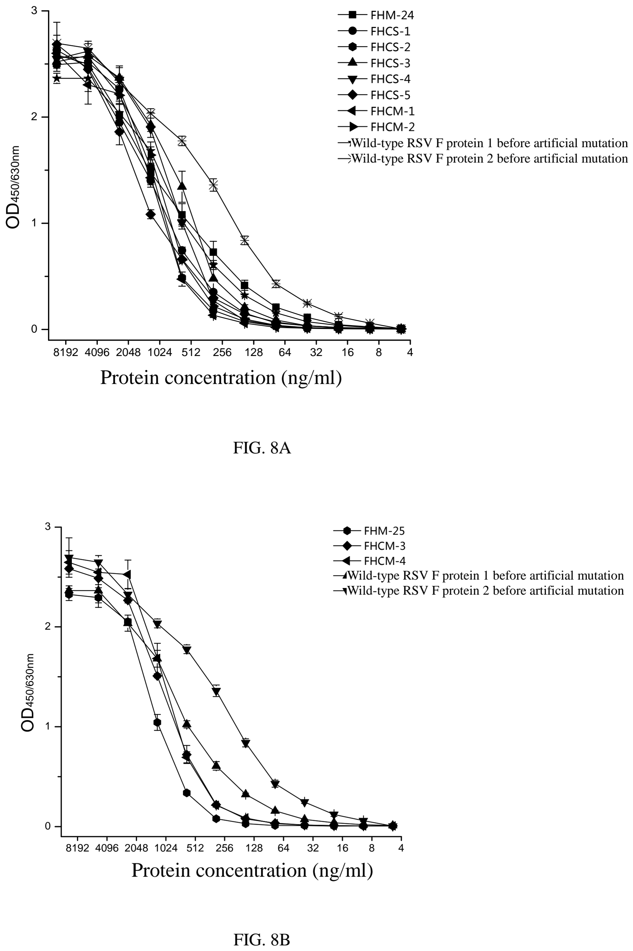

FIG. 8A is a comparison diagram of results of Synagis^{®} binding experiments of mutants FHCS-1, FHCS-2, FHCS-3, FHCS-4, FHCS-5, FHCM-1 and FHCM-2 constructed in an example of the present disclosure, a mutant FHM-24, a wild-type RSV F protein 1 before artificial mutation, and a wild-type RSV F protein 2 before artificial mutation; and FIG. 8B is a comparison diagram of results of Synagis^{®} binding experiments of mutants FHCM-3 and FHCM-4 constructed in an example of the present disclosure, a mutant FHM-25, a wild-type RSV F protein 1 before artificial mutation, and a wild-type RSV F protein 2 before artificial mutation; and

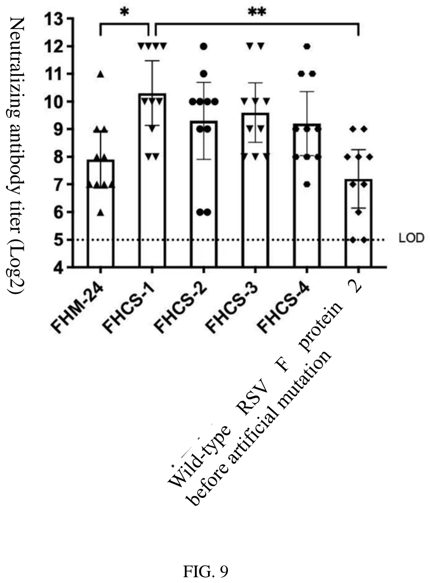

FIG. 9 is a comparison diagram of detection results of live RSV strain A2 neutralizing antibodies of mutants FHCS-1, FHCS-2, FHCS-3 and FHCS-4 constructed in an example of the present disclosure, a mutant FHM-24, and a wild-type RSV F protein 2 before artificial mutation.

### Sequence table

Illustrative sequences provided in the present disclosure are shown in Tables 1 to 3.

**Table 1. Amino acid sequence of wild-type RSV F protein**

| Wild-type sequence name and descriptio n | SEQ ID No. | Amino acid sequence |
|---|---|---|
| Full-lengt h amino acid sequence of F protein of RSV strain A2 (Ref: McLellan J. S., et al. Science, 2013, 342:592-5 98) | 1 | MELLILKANAITTILTAVTFCFASGQNITEEFYQSTCSAVS KGYLSALRTGWYTSVITIELSNIKENKCNGTDAKVKLIK QELDKYKNAVTELQLLMQSTPATNNRARRELPRFMNYT LNNAKKTNVTLSKKRKRRFLGFLLGVGSAIASGVAVSK VLHLEGEVNKIKSALLSTNKAVVSLSNGVSVLTSKVLDL KNYIDKQLLPIVNKQSCSISNIETVIEFQQKNNRLLEITRE FSVNAGVTTPVSTYMLTNSELLSLINDMPITNDQKKLMS NNVQIVRQQSYSIMSIIKEEVLAYVVQLPLYGVIDTPCW KLHTSPLCTTNTKEGSNICLTRTDRGWYCDNAGSVSFFP QAETCKVQSNRVFCDTMNSLTLPSEVNLCNVDIFNPKY DCKIMTSKTDVSSSVITSLGAIVSCYGKTKCTASNKNRGI IKTFSNGCDYVSNKGVDTVSVGNTLYYVNKQEGKSLYV KGEPIINFYDPLVFPSDEFDASISQVNEKINQSLAFIRKSD ELLHNVNAGKSTTNIMITTIIIVIIVILLSLIAVGLLLYCKA RSTPVTLSKDQLSGINNIAFSN |
| Full-lengt h amino acid sequence of F | 2 | MELLILKANAITTILTAVTFCFASGQNITEEFYQSTCSAVS KGYLSALRTGWYTSVITIELSNIKENKCNGTDAKVKLIK QELDKYKNAVTELQLLMQSTPPTNNRARRELPRFMNYT LNNAKKTNVTLSKKRKRRFLGFLLGVGSAIASGVAVSK VLHLEGEVNKIKSALLSTNKAVVSLSNGVSVLTSKVLDL |
| protein of RSV strain A2 (UniProtK B/Swiss-P rot: P03420.1) | | KNYIDKQLLPIVNKQSCSISNIETVIEFQQKNNRLLEITRE FSVNAGVTTPVSTYMLTNSELLSLINDMPITNDQKKLMS NNVQIVRQQSYSIMSIIKEEVLAYVVQLPLYGVIDTPCW KLHTSPLCTTNTKEGSNICLTRTDRGWYCDNAGSVSFFP QAETCKVQSNRVFCDTMNSLTLPSEINLCNVDIFNPKYD CKIMTSKTDVSSSVITSLGAIVSCYGKTKCTASNKNRGII KTFSNGCDYVSNKGMDTVSVGNTLYYVNKQEGKSLYV KGEPIINFYDPLVFPSDEFDASISQVNEKINQSLAFIRKSD ELLHNVNAGKSTTNIMITTIIIVIIVILLSLIAVGLLLYCKA RSTPVTLSKDQLSGINNIAFSN |
| Full-lengt h amino acid sequence of F protein of RSV strain ON1 of subtype A (GenBank: QYW1142 7.1) | 3 | MELPIIKTNAITAILAAVTLCFASSQNITEEFYQSTCSAVS KGYLSALRTGWYTSVITIELSNIKENKCNGTDAKVKLIK QELDKYKNAVTELQLLMQSTPAANSRARRELPRFMNYT LNNTKNTNVTLSKKRKRRFLGFLLGVGSAIASGIAVSKV LHLEGEVNKIKSALLSTNKAVVSLSNGVSVLTSKVLDLK NYIDKQLLPIVNKQSCSISNIETVIEFQQKNNRLLEITREF SVNAGVTTPVSTYMLTNSELLSLINDMPITNDQKKLMSS NVQIVRQQSYSIMSIIKEEVLAYVVQLPLYGVIDTPCWK LHTSPLCTTNTKEGSNICLTRTDRGWYCDNAGSVSFFPQ AETCKVQSNRVFCDTMNSLTLPSEVNLCNIDIFNPKYDC KIMTSKTDVSSSVITSLGAIVSCYGKTKCTASNKNRGIIK TFSNGCDYVSNKGVDTVSVGNTLYYVNKQEGKSLYVK GEPIINFYDPLVFPSDEFDASISQVNEKINQSLAFIRKSDE LLHNVNAGKSTTNIMITTIIIVIIVILLALIAVGLLLYCKAR STPVTLSKDQLSGINNIAFSN |
| Full-lengt h amino acid sequence of F protein of RSV strain BA of subtype B (GenBank: QYW1294 0.1) | 4 | MELLIHRSSAIFLTLAINALHLTSSQNITEEFYQSTCSAVS RGYLSALRTGWYTSVITIELSNIKETKCNGTDTKVKLIK QELDKYKNAVTELQLLMQNTPAVNNRARREAPQYMNY TINTTKNLNVSISKKRKRRFLGFLLGVGSAIASGIAVSKV LHLEGEVNKIKNALQLTNKAVVSLSNGVSVLTSRVLDLK NYINNQLLPMVNRQSCRISNIETVIEFQQKNSRLLEITRE FSVNAGVTTPLSTYMLTNSELLSLINDMPITNDQKKLMS SNVQIVRQQSYSIMSIIKEEVLAYVVQLPIYGVIDTPCWK LHTSPLCTTNIKEGSNICLTRTDRGWYCDNAGSVSFFPQ ADTCKVQSNRVFCDTMNSLTLPSEVSLCNTDIFNSKYDC KIMTSKTDISSSVITSLGAIVSCYGKTKCTASNKNRGIIKT FSNGCDYVSNKGVDTVSVGNTLYYVNKLEGKNLYVKG EPIINYYDPLVFPSDEFDASISQVNEKINQSLAFIRRSDEL LHNVNTGKSTTNIMITAIIIVIIVVLLSLIAIGLLLYCKAKN TPVTLSKDQLSGINNIAFSK |
| Full-lengt h amino acid sequence of F protein of RSV of subtype B (GenBank: AHV8075 | 5 | MELLIHRSSAIFLTLAINALYLTSSQNITEEFYQSTCSAVS RGYFSALRTGWYTSVITIELSNIKETKCNGTDTKVKLIK QELDKYKNAVTELQLLMQNTPAANNRARREAPQYMNY TINTTKNLNVSISKKRKRRFLGFLLGVGSAIASGIAVSKV LHLEGEVNKIKNALLSTNKAVVSLSNGVSVLTSKVLDLK NYINNQLLPIVNQQSCRISNIETVIEFQQKNSRLLEITREF SVNAGVTTPLSTYMLTNSELLSLINDMPITNDQKKLMSS NVQIVRQQSYSIMSIIKEEVLAYVVQLPIYGVIDTPCWKL HTSPLCTTNIKEGSNICLTRTDRGWYCDNAGSVSFFPQA DTCKVQSNRVFCDTMNSLTLPSEVSLCNTDIFNSKYDCK |
| 8) | | IMTSKTDISSSVITSLGAIVSCYGKTKCTASNKNRGIIKTF SNGCDYVSNKGVDTVSVGNTLYYVNKLEGKNLYVKGE PIINYYDPLVFPSDEFDASISQVNEKINQSLAFIRRSDELL HNVNTGKSTTNIMITAIIIVIIVVLLSLIAIGLLLYCKAKNT PVTLSKDQLSGINNIAFSK |
| Full-lengt h amino acid sequence of F protein of RSV of subtype B (UniProtK B/Swiss-P rot: P13843.1) | 6 | MELLIHRSSAIFLTLAVNALYLTSSQNITEEFYQSTCSAVS RGYFSALRTGWYTSVITIELSNIKETKCNGTDTKVKLIK QELDKYKNAVTELQLLMQNTPAANNRARREAPQYMNY TINTTKNLNVSISKKRKRRFLGFLLGVGSAIASGIAVSKV LHLEGEVNKIKNALLSTNKAVVSLSNGVSVLTSKVLDLK NYINNRLLPIVNQQSCRISNIETVIEFQQMNSRLLEITREF SVNAGVTTPLSTYMLTNSELLSLINDMPITNDQKKLMSS NVQIVRQQSYSIMSIIKEEVLAYVVQLPIYGVIDTPCWKL HTSPLCTTNIKEGSNICLTRTDRGWYCDNAGSVSFFPQA DTCKVQSNRVFCDTMNSLTLPSEVSLCNTDIFNSKYDCK IMTSKTDISSSVITSLGAIVSCYGKTKCTASNKNRGIIKTF SNGCDYVSNKGVDTVSVGNTLYYVNKLEGKNLYVKGE PIINYYDPLVFPSDEFDASISQVNEKINQSLAFIRRSDELL HNVNTGKSTTNIMITTIIIVIIVVLLSLIAIGLLLYCKAKNT PVTLSKDQLSGINNIAFSK |

**Table 2. Amino acid sequence of wild-type RSV F protein before artificial mutation**

| Sequence name and descriptio n | SEQ ID No. | Amino acid sequence |
|---|---|---|
| F protein of RSV strain A2 (SEQ ID No. 1): an F0 extracellul ar region (with amino acids at positions 514 to 574 removed at a C-terminal ), and a signal peptide removed | 7 | QNITEEFYQSTCSAVSKGYLSALRTGWYTSVITIELSNIK ENKCNGTDAKVKLIKQELDKYKNAVTELQLLMQSTPAT NNRARRELPRFMNYTLNNAKKTNVTLSKKRKRRFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNVDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELL |
| F protein of RSV strain A2 (SEQ ID No. 1): an F0 extracellul ar region (with amino acids at positions 530 to 574 removed at a C-terminal ), and a signal peptide removed | 8 | QNITEEFYQSTCSAVSKGYLSALRTGWYTSVITIELSNIK ENKCNGTDAKVKLIKQELDKYKNAVTELQLLMQSTPAT NNRARRELPRFMNYTLNNAKKTNVTLSKKRKRRFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNVDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELLHNVNAGKSTTNIMITT |
| F protein of RSV strain A2 (SEQ ID No. 1): an F0 extracellul ar region (with amino acids at positions 514 to 574 removed at a C-terminal ), a signal peptide removed, mutation of a cleavage site, and p27 | 9 | QNITEEFYQSTCSAVSKGYLSALRTGWYTSVITIELSNIK ENKCNGTDAKVKLIKQELDKYKNAVTELQLLMQSTPAT NNQARRELPRFMNYTLNNAKKTNVTLSKKRKSSFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNVDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELL |
| F protein of RSV strain A2 (SEQ ID No. 1): an F0 extracellul ar region (with amino acids at positions 514 to 574 removed at a C-terminal ), a signal peptide removed, a combinati on of an independe nt F1 and an independe nt F2, and p27 artificially removed | 10, 11 | 10):QNITEEFYQSTCSAVSKGYLSALRTGWYTSVITIELS NIKENKCNGTDAKVKLIKQELDKYKNAVTELQLLMQST PATNNRARR FLGFLLGVGSAIASGVAVSKVLHLEGEVNKIKSALLSTN KAVVSLSNGVSVLTSKVLDLKNYIDKQLLPIVNKQSCSIS NIETVIEFQQKNNRLLEITREFSVNAGVTTPVSTYMLTNS ELLSLINDMPITNDQKKLMSNNVQIVRQQSYSIMSIIKEE VLAYVVQLPLYGVIDTPCWKLHTSPLCTTNTKEGSNICL TRTDRGWYCDNAGSVSFFPQAETCKVQSNRVFCDTMN F2 polypeptide(SEQ ID No. F1 polypeptide(SEQ ID No. 11): SLTLPSEVNLCNVDIFNPKYDCKIMTSKTDVSSSVITSLG AIVSCYGKTKCTASNKNRGIIKTFSNGCDYVSNKGVDT VSVGNTLYYVNKQEGKSLYVKGEPIINFYDPLVFPSDEF DASISQVNEKINQSLAFIRKSDELL |
| F protein of RSV strain A2 (SEQ ID No. 1): an F0 extracellul ar region (with amino acids at positions 514 to 574 removed at a C-terminal ), a signal peptide removed, F2-linker-F1, and a linker replacing p27 | 12 | QNITEEFYQSTCSAVSKGYLSALRTGWYTSVITIELSNIK ENKCNGTDAKVKLIKQELDKYKNAVTELQLLMQSTPAT NNQARGSGSGRSLGFLLGVGSAIASGVAVSKVLHLEGE VNKIKSALLSTNKAVVSLSNGVSVLTSKVLDLKNYIDKQ LLPIVNKQSCSISNIETVIEFQQKNNRLLEITREFSVNAGV TTPVSTYMLTNSELLSLINDMPITNDQKKLMSNNVQIVR QQSYSIMSIIKEEVLAYVVQLPLYGVIDTPCWKLHTSPLC TTNTKEGSNICLTRTDRGWYCDNAGSVSFFPQAETCKV QSNRVFCDTMNSLTLPSEVNLCNVDIFNPKYDCKIMTSK TDVSSSVITSLGAIVSCYGKTKCTASNKNRGIIKTFSNGC DYVSNKGVDTVSVGNTLYYVNKQEGKSLYVKGEPIINF YDPLVFPSDEFDASISQVNEKINQSLAFIRKSDELL |
| F protein of RSV strain A2 (SEQ ID No. 1): an F0 extracellular region (with amino acids at positions 514 to 574 removed at a C-terminal ), a signal peptide removed, F2-linker-F1, a linker replacing p27, and F2 and F1 with more amino acids removed | 13 | QNITEEFYQSTCSAVSKGYLSALRTGWYTSVITIELSNIK ENKCNGTDAKVKLIKQELDKYKNAVTELQLLMQSTPAT GSGSAIASGVAVSKVLHLEGEVNKIKSALLSTNKAVVSL SNGVSVLTSKVLDLKNYIDKQLLPIVNKQSCSISNIETVIE FQQKNNRLLEITREFSVNAGVTTPVSTYMLTNSELLSLIN DMPITNDQKKLMSNNVQIVRQQSYSIMSIIKEEVLAYVV QLPLYGVIDTPCWKLHTSPLCTTNTKEGSNICLTRTDRG WYCDNAGSVSFFPQAETCKVQSNRVFCDTMNSLTLPSE VNLCNVDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELL |
| F protein of RSV strain A2 (SEQ ID No. 2): an F0 extracellul ar region (with amino acids at positions 514 to 574 removed at a C-terminal ), and a signal peptide removed | 14 | QNITEEFYQSTCSAVSKGYLSALRTGWYTSVITIELSNIK ENKCNGTDAKVKLIKQELDKYKNAVTELQLLMQSTPPT NNRARRELPRFMNYTLNNAKKTNVTLSKKRKRRFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EINLCNVDIFNPKYDCKIMTSKTDVSSSVITSLGAIVSCY GKTKCTASNKNRGIIKTFSNGCDYVSNKGMDTVSVGNT LYYVNKQEGKSLYVKGEPIINFYDPLVFPSDEFDASISQV NEKINQSLAFIRKSDELL |
| F protein of RSV strain A2 (SEQ ID No. 2): an F0 extracellul ar region (with amino acids at positions 530 to 574 removed at a C-terminal ), and a signal peptide removed | 15 | QNITEEFYQSTCSAVSKGYLSALRTGWYTSVITIELSNIK ENKCNGTDAKVKLIKQELDKYKNAVTELQLLMQSTPPT NNRARRELPRFMNYTLNNAKKTNVTLSKKRKRRFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EINLCNVDIFNPKYDCKIMTSKTDVSSSVITSLGAIVSCY GKTKCTASNKNRGIIKTFSNGCDYVSNKGMDTVSVGNT LYYVNKQEGKSLYVKGEPIINFYDPLVFPSDEFDASISQV NEKINQSLAFIRKSDELLHNVNAGKSTTNIMITT |
| F protein of RSV strain A2 (SEQ ID No. 2): an F0 extracellul ar region (with amino acids at positions 514 to 574 removed at a C-terminal ), a signal peptide removed, mutation of a cleavage site, and p27 | 16 | QNITEEFYQSTCSAVSKGYLSALRTGWYTSVITIELSNIK ENKCNGTDAKVKLIKQELDKYKNAVTELQLLMQSTPPT NNQARRELPRFMNYTLNNAKKTNVTLSKKRKSSFLGFL LGVGSAIASGVAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSNNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EINLCNVDIFNPKYDCKIMTSKTDVSSSVITSLGAIVSCY GKTKCTASNKNRGIIKTFSNGCDYVSNKGMDTVSVGNT LYYVNKQEGKSLYVKGEPIINFYDPLVFPSDEFDASISQV NEKINQSLAFIRKSDELL |
| F protein of RSV strain A2 (SEQ ID No. 2): an F0 extracellul ar region (with amino acids at positions 514 to 574 removed at a C-terminal ), a signal peptide removed, a combinati on of an independe nt F1 and an independe nt F2, and p27 artificially removed | 17, 18 | QNITEEFYQSTCSAVSKGYLSALRTGWYTSVITIELSNIK ENKCNGTDAKVKLIKQELDKYKNAVTELQLLMQSTPPT NNRARR FLGFLLGVGSAIASGVAVSKVLHLEGEVNKIKSALLSTN KAVVSLSNGVSVLTSKVLDLKNYIDKQLLPIVNKQSCSIS NIETVIEFQQKNNRLLEITREFSVNAGVTTPVSTYMLTNS ELLSLINDMPITNDQKKLMSNNVQIVRQQSYSIMSIIKEE VLAYVVQLPLYGVIDTPCWKLHTSPLCTTNTKEGSNICL TRTDRGWYCDNAGSVSFFPQAETCKVQSNRVFCDTMN SLTLPSEINLCNVDIFNPKYDCKIMTSKTDVSSSVITSLGA IVSCYGKTKCTASNKNRGIIKTFSNGCDYVSNKGMDTV SVGNTLYYVNKQEGKSLYVKGEPIINFYDPLVFPSDEFD F2 polypeptide(SEQ ID No. 17): F1 polypeptide(SEQ ID No. 18): ASISQVNEKINQSLAFIRKSDELL |
| F protein of RSV strain A2 (SEQ ID No. 2): an F0 extracellul ar region (with amino acids at positions 514 to 574 removed at a C-terminal ), a signal peptide removed, F2-linker-F1, and a linker replacing p27 | 19 | QNITEEFYQSTCSAVSKGYLSALRTGWYTSVITIELSNIK ENKCNGTDAKVKLIKQELDKYKNAVTELQLLMQSTPPT NNQARGSGSGRSLGFLLGVGSAIASGVAVSKVLHLEGE VNKIKSALLSTNKAVVSLSNGVSVLTSKVLDLKNYIDKQ LLPIVNKQSCSISNIETVIEFQQKNNRLLEITREFSVNAGV TTPVSTYMLTNSELLSLINDMPITNDQKKLMSNNVQIVR QQSYSIMSIIKEEVLAYVVQLPLYGVIDTPCWKLHTSPLC TTNTKEGSNICLTRTDRGWYCDNAGSVSFFPQAETCKV QSNRVFCDTMNSLTLPSEINLCNVDIFNPKYDCKIMTSK TDVSSSVITSLGAIVSCYGKTKCTASNKNRGIIKTFSNGC DYVSNKGMDTVSVGNTLYYVNKQEGKSLYVKGEPIINF YDPLVFPSDEFDASISQVNEKINQSLAFIRKSDELL |
| F protein of RSV strain A2 (SEQ ID No. 2): an F0 extracellul ar region (with amino acids at positions 514 to 574 removed at a C-terminal ), a signal peptide removed, F2-linker-F1, a linker replacing p27, and F2 and F1 with more amino acids removed | 20 | QNITEEFYQSTCSAVSKGYLSALRTGWYTSVITIELSNIK ENKCNGTDAKVKLIKQELDKYKNAVTELQLLMQSTPPT GSGSAIASGVAVSKVLHLEGEVNKIKSALLSTNKAVVSL SNGVSVLTSKVLDLKNYIDKQLLPIVNKQSCSISNIETVIE FQQKNNRLLEITREFSVNAGVTTPVSTYMLTNSELLSLIN DMPITNDQKKLMSNNVQIVRQQSYSIMSIIKEEVLAYVV QLPLYGVIDTPCWKLHTSPLCTTNTKEGSNICLTRTDRG WYCDNAGSVSFFPQAETCKVQSNRVFCDTMNSLTLPSEI NLCNVDIFNPKYDCKIMTSKTDVSSSVITSLGAIVSCYG KTKCTASNKNRGIIKTFSNGCDYVSNKGMDTVSVGNTL YYVNKQEGKSLYVKGEPIINFYDPLVFPSDEFDASISQVN EKINQSLAFIRKSDELL |
| F protein of RSV strain ON1 of subtype A (SEQ ID No. 3): an F0 extracellul ar region (with amino acids at positions 514 to 574 removed at a C-terminal ), and a signal peptide removed | 21 | QNITEEFYQSTCSAVSKGYLSALRTGWYTSVITIELSNIK ENKCNGTDAKVKLIKQELDKYKNAVTELQLLMQSTPAA NSRARRELPRFMNYTLNNTKNTNVTLSKKRKRRFLGFL LGVGSAIASGIAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSSNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNIDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELL |
| F protein of RSV strain ON1 of subtype A (SEQ ID No. 3): an F0 extracellul ar region (with amino acids at positions 530 to 574 removed at a C-terminal ), and a signal peptide removed | 22 | QNITEEFYQSTCSAVSKGYLSALRTGWYTSVITIELSNIK ENKCNGTDAKVKLIKQELDKYKNAVTELQLLMQSTPAA NSRARRELPRFMNYTLNNTKNTNVTLSKKRKRRFLGFL LGVGSAIASGIAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSSNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNIDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELLHNVNAGKSTTNIMITT |
| F protein of RSV strain ON1 of subtype A (SEQ ID No. 3): an F0 extracellul ar region (with amino acids at positions 514 to 574 removed at a C-terminal ), a signal peptide removed, mutation of a cleavage site, and p27 | 23 | QNITEEFYQSTCSAVSKGYLSALRTGWYTSVITIELSNIK ENKCNGTDAKVKLIKQELDKYKNAVTELQLLMQSTPAA NSQARRELPRFMNYTLNNTKNTNVTLSKKRKSSFLGFL LGVGSAIASGIAVSKVLHLEGEVNKIKSALLSTNKAVVS LSNGVSVLTSKVLDLKNYIDKQLLPIVNKQSCSISNIETVI EFQQKNNRLLEITREFSVNAGVTTPVSTYMLTNSELLSLI NDMPITNDQKKLMSSNVQIVRQQSYSIMSIIKEEVLAYV VQLPLYGVIDTPCWKLHTSPLCTTNTKEGSNICLTRTDR GWYCDNAGSVSFFPQAETCKVQSNRVFCDTMNSLTLPS EVNLCNIDIFNPKYDCKIMTSKTDVSSSVITSLGAIVSCY GKTKCTASNKNRGIIKTFSNGCDYVSNKGVDTVSVGNT LYYVNKQEGKSLYVKGEPIINFYDPLVFPSDEFDASISQV NEKINQSLAFIRKSDELL |
| F protein of RSV strain ON1 of subtype A (SEQ ID No. 3): an F0 extracellul ar region (with amino acids at positions 514 to 574 removed at a C-terminal ), a signal peptide removed, a combinati on of an independe nt F1 and an independe nt F2, and p27 artificially removed | 24, 25 | QNITEEFYQSTCSAVSKGYLSALRTGWYTSVITIELSNIK ENKCNGTDAKVKLIKQELDKYKNAVTELQLLMQSTPAA NSRARR FLGFLLGVGSAIASGIAVSKVLHLEGEVNKIKSALLSTNK AVVSLSNGVSVLTSKVLDLKNYIDKQLLPIVNKQSCSISN IETVIEFQQKNNRLLEITREFSVNAGVTTPVSTYMLTNSE LLSLINDMPITNDQKKLMSSNVQIVRQQSYSIMSIIKEEV LAYVVQLPLYGVIDTPCWKLHTSPLCTTNTKEGSNICLT RTDRGWYCDNAGSVSFFPQAETCKVQSNRVFCDTMNSL TLPSEVNLCNIDIFNPKYDCKIMTSKTDVSSSVITSLGAIV SCYGKTKCTASNKNRGIIKTFSNGCDYVSNKGVDTVSV GNTLYYVNKQEGKSLYVKGEPIINFYDPLVFPSDEFDASI F2 polypeptide(SEQ ID No. 24): F1 polypeptide(SEQ ID No. 25): SQVNEKINQSLAFIRKSDELL |
| F protein of RSV strain ON1 of subtype A (SEQ ID No. 3): an F0 extracellular region (with amino acids at positions 514 to 574 removed at a C-terminal ), a signal peptide removed, F2-linker-F1, and a linker replacing p27 | 26 | QNITEEFYQSTCSAVSKGYLSALRTGWYTSVITIELSNIK ENKCNGTDAKVKLIKQELDKYKNAVTELQLLMQSTPAA NSQARGSGSGRSLGFLLGVGSAIASGIAVSKVLHLEGEV NKIKSALLSTNKAVVSLSNGVSVLTSKVLDLKNYIDKQL LPIVNKQSCSISNIETVIEFQQKNNRLLEITREFSVNAGVT TPVSTYMLTNSELLSLINDMPITNDQKKLMSSNVQIVRQ QSYSIMSIIKEEVLAYVVQLPLYGVIDTPCWKLHTSPLCT TNTKEGSNICLTRTDRGWYCDNAGSVSFFPQAETCKVQ SNRVFCDTMNSLTLPSEVNLCNIDIFNPKYDCKIMTSKT DVSSSVITSLGAIVSCYGKTKCTASNKNRGIIKTFSNGCD YVSNKGVDTVSVGNTLYYVNKQEGKSLYVKGEPIINFY DPLVFPSDEFDASISQVNEKINQSLAFIRKSDELL |
| F protein of RSV strain ON1 of subtype A (SEQ ID No. 3): an F0 extracellular region (with amino acids at positions 514 to 574 removed at a C-terminal ), a signal peptide removed, F2-linker-F1, a linker replacing p27, and F2 and F1 with more amino acids removed | 27 | QNITEEFYQSTCSAVSKGYLSALRTGWYTSVITIELSNIK ENKCNGTDAKVKLIKQELDKYKNAVTELQLLMQSTPAA GSGSAIASGIAVSKVLHLEGEVNKIKSALLSTNKAVVSLS NGVSVLTSKVLDLKNYIDKQLLPIVNKQSCSISNIETVIEF QQKNNRLLEITREFSVNAGVTTPVSTYMLTNSELLSLIN DMPITNDQKKLMSSNVQIVRQQSYSIMSIIKEEVLAYVV QLPLYGVIDTPCWKLHTSPLCTTNTKEGSNICLTRTDRG WYCDNAGSVSFFPQAETCKVQSNRVFCDTMNSLTLPSE VNLCNIDIFNPKYDCKIMTSKTDVSSSVITSLGAIVSCYG KTKCTASNKNRGIIKTFSNGCDYVSNKGVDTVSVGNTL YYVNKQEGKSLYVKGEPIINFYDPLVFPSDEFDASISQVN EKINQSLAFIRKSDELL |
| F protein of RSV strain BA of subtype B (SEQ ID No. 4): an F0 extracellul ar region (with amino acids at positions 514 to 574 removed at a C-terminal ), and a signal peptide removed | 28 | QNITEEFYQSTCSAVSRGYLSALRTGWYTSVITIELSNIK ETKCNGTDTKVKLIKQELDKYKNAVTELQLLMQNTPAV NNRARREAPQYMNYTINTTKNLNVSISKKRKRRFLGFL LGVGSAIASGIAVSKVLHLEGEVNKIKNALQLTNKAVVS LSNGVSVLTSRVLDLKNYINNQLLPMVNRQSCRISNIET VIEFQQKNSRLLEITREFSVNAGVTTPLSTYMLTNSELLS LINDMPITNDQKKLMSSNVQIVRQQSYSIMSIIKEEVLAY VVQLPIYGVIDTPCWKLHTSPLCTTNIKEGSNICLTRTDR GWYCDNAGSVSFFPQADTCKVQSNRVFCDTMNSLTLPS EVSLCNTDIFNSKYDCKIMTSKTDISSSVITSLGAIVSCYG KTKCTASNKNRGIIKTFSNGCDYVSNKGVDTVSVGNTL YYVNKLEGKNLYVKGEPIINYYDPLVFPSDEFDASISQV NEKINQSLAFIRRSDELL |
| F protein of RSV strain BA of subtype B (SEQ ID No. 4): an F0 extracellul ar region (with amino acids at positions 530 to 574 removed at a C-terminal ), and a signal peptide removed | 29 | QNITEEFYQSTCSAVSRGYLSALRTGWYTSVITIELSNIK ETKCNGTDTKVKLIKQELDKYKNAVTELQLLMQNTPAV NNRARREAPQYMNYTINTTKNLNVSISKKRKRRFLGFL LGVGSAIASGIAVSKVLHLEGEVNKIKNALQLTNKAVVS LSNGVSVLTSRVLDLKNYINNQLLPMVNRQSCRISNIET VIEFQQKNSRLLEITREFSVNAGVTTPLSTYMLTNSELLS LINDMPITNDQKKLMSSNVQIVRQQSYSIMSIIKEEVLAY VVQLPIYGVIDTPCWKLHTSPLCTTNIKEGSNICLTRTDR GWYCDNAGSVSFFPQADTCKVQSNRVFCDTMNSLTLPS EVSLCNTDIFNSKYDCKIMTSKTDISSSVITSLGAIVSCYG KTKCTASNKNRGIIKTFSNGCDYVSNKGVDTVSVGNTL YYVNKLEGKNLYVKGEPIINYYDPLVFPSDEFDASISQV NEKINQSLAFIRRSDELLHNVNTGKSTTNIMITA |
| F protein of RSV strain BA of subtype B (SEQ ID No. 4): an F0 extracellul ar region (with amino acids at positions 514 to 574 removed at a C-terminal ), a signal peptide removed, mutation of a cleavage site, and p27 | 30 | QNITEEFYQSTCSAVSRGYLSALRTGWYTSVITIELSNIK ETKCNGTDTKVKLIKQELDKYKNAVTELQLLMQNTPAV NNQARREAPQYMNYTINTTKNLNVSISKKRKSSFLGFLL GVGSAIASGIAVSKVLHLEGEVNKIKNALQLTNKAVVSL SNGVSVLTSRVLDLKNYINNQLLPMVNRQSCRISNIETVI EFQQKNSRLLEITREFSVNAGVTTPLSTYMLTNSELLSLI NDMPITNDQKKLMSSNVQIVRQQSYSIMSIIKEEVLAYV VQLPIYGVIDTPCWKLHTSPLCTTNIKEGSNICLTRTDRG WYCDNAGSVSFFPQADTCKVQSNRVFCDTMNSLTLPSE VSLCNTDIFNSKYDCKIMTSKTDISSSVITSLGAIVSCYG KTKCTASNKNRGIIKTFSNGCDYVSNKGVDTVSVGNTL YYVNKLEGKNLYVKGEPIINYYDPLVFPSDEFDASISQV NEKINQSLAFIRRSDELL |
| F protein of RSV strain BA of subtype B (SEQ ID No. 4): an F0 extracellul ar region (with amino acids at positions 514 to 574 removed at a C-terminal ), a signal peptide removed, a combinati on of an independe nt F1 and an independe nt F2, and p27 artificially removed | 31, 32 | QNITEEFYQSTCSAVSRGYLSALRTGWYTSVITIELSNIK ETKCNGTDTKVKLIKQELDKYKNAVTELQLLMQNTPAV NNRARR FLGFLLGVGSAIASGIAVSKVLHLEGEVNKIKNALQLTN KAVVSLSNGVSVLTSRVLDLKNYINNQLLPMVNRQSCRI SNIETVIEFQQKNSRLLEITREFSVNAGVTTPLSTYMLTN SELLSLINDMPITNDQKKLMSSNVQIVRQQSYSIMSIIKE EVLAYVVQLPIYGVIDTPCWKLHTSPLCTTNIKEGSNICL TRTDRGWYCDNAGSVSFFPQADTCKVQSNRVFCDTMN F2 polypeptide(SEQ ID No. 31): F1 polypeptide(SEQ ID No. 32): SLTLPSEVSLCNTDIFNSKYDCKIMTSKTDISSSVITSLGAI VSCYGKTKCTASNKNRGIIKTFSNGCDYVSNKGVDTVS VGNTLYYVNKLEGKNLYVKGEPIINYYDPLVFPSDEFDA SISQVNEKINQSLAFIRRSDELL |
| F protein of RSV strain BA of subtype B (SEQ ID No. 4): an F0 extracellul ar region (with amino acids at positions 514 to 574 removed at a C-terminal ), a signal peptide removed, F2-linker-F1, and a linker replacing p27 | 33 | QNITEEFYQSTCSAVSRGYLSALRTGWYTSVITIELSNIK ETKCNGTDTKVKLIKQELDKYKNAVTELQLLMQNTPAV NNQARGSGSGRSLGFLLGVGSAIASGIAVSKVLHLEGEV NKIKNALQLTNKAVVSLSNGVSVLTSRVLDLKNYINNQL LPMVNRQSCRISNIETVIEFQQKNSRLLEITREFSVNAGV TTPLSTYMLTNSELLSLINDMPITNDQKKLMSSNVQIVR QQSYSIMSIIKEEVLAYVVQLPIYGVIDTPCWKLHTSPLC TTNIKEGSNICLTRTDRGWYCDNAGSVSFFPQADTCKVQ SNRVFCDTMNSLTLPSEVSLCNTDIFNSKYDCKIMTSKT DISSSVITSLGAIVSCYGKTKCTASNKNRGIIKTFSNGCD YVSNKGVDTVSVGNTLYYVNKLEGKNLYVKGEPIINYY DPLVFPSDEFDASISQVNEKINQSLAFIRRSDELL |
| F protein of RSV strain BA of subtype B (SEQ ID No. 4): an F0 extracellul ar region (with amino acids at positions 514 to 574 removed at a C-terminal ), a signal peptide removed, F2-linker-F1, a linker replacing p27, and F2 and F1 with more amino acids removed | 34 | QNITEEFYQSTCSAVSRGYLSALRTGWYTSVITIELSNIK ETKCNGTDTKVKLIKQELDKYKNAVTELQLLMQNTPAV GSGSAIASGIAVSKVLHLEGEVNKIKNALQLTNKAVVSL SNGVSVLTSRVLDLKNYINNQLLPMVNRQSCRISNIETVI EFQQKNSRLLEITREFSVNAGVTTPLSTYMLTNSELLSLI NDMPITNDQKKLMSSNVQIVRQQSYSIMSIIKEEVLAYV VQLPIYGVIDTPCWKLHTSPLCTTNIKEGSNICLTRTDRG WYCDNAGSVSFFPQADTCKVQSNRVFCDTMNSLTLPSE VSLCNTDIFNSKYDCKIMTSKTDISSSVITSLGAIVSCYG KTKCTASNKNRGIIKTFSNGCDYVSNKGVDTVSVGNTL YYVNKLEGKNLYVKGEPIINYYDPLVFPSDEFDASISQV NEKINQSLAFIRRSDELL |

**Table 3 Illustrative amino acid sequence of mutant of wild-type RSV F protein (after artificial mutation)**

| Mut ant ID | SE Q ID No. | Intro duced mutation | Amino acid sequence |
|---|---|---|---|
| FHS-1 | 35 | 227N→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-2 | 36 | 227N→G | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKGNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-3 | 37 | 227N→A | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKANRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-4 | 38 | 227N→V | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKVNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-5 | 39 | 227N→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKLNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-6 | 40 | 227N→I | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKINRLLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-7 | 41 | 227N→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKWNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-8 | 42 | 227N→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKFNRLLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-9 | 43 | 227N→P | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKPNRLLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-10 | 44 | 227N→S | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKSNRLLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-11 | 45 | 227N→T | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKTNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-12 | 46 | 227→C | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKCNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-13 | 47 | 227N→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKYNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-14 | 48 | 227N→Q | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKQNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-15 | 49 | 227N→D | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKDNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-16 | 50 | 227N→E | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKENRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-17 | 51 | 227N→K | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKKNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-18 | 52 | 227N→R | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKRNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-19 | 53 | 227N→H | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKHNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-20 | 54 | 230L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRMLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-21 | 55 | 230L→G | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRGLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-22 | 56 | 230L→A | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRALEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-23 | 57 | 230L→V | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRVLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-24 | 58 | 230L→I | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRILEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-25 | 59 | 230L→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRWLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-26 | 60 | 230L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-27 | 61 | 230L→P | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRPLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-28 | 62 | 230L→S | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRSLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-29 | 63 | 230L→T | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRTLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-30 | 64 | 230L→C | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRCLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-31 | 65 | 230L→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRYLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-32 | 66 | 230L→Q | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRQLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-33 | 67 | 230L→N | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRNLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-34 | 68 | 230L→D | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRDLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-35 | 69 | 230L→E | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRELEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-36 | 70 | 230L→K | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRKLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-37 | 71 | 230L→R | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRRLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-38 | 72 | 230L→H | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRHLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-39 | 73 | 234T→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEILR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-40 | 74 | 234T→I | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIIRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-41 | 75 | 234T→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIMR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-42 | 76 | 234T→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIFR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-43 | 77 | 234T→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIYR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-44 | 78 | 234T→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIWR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-45 | 79 | 238S→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFLVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-46 | 80 | 238S→I | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFIVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-47 | 81 | 238S→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFMVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-48 | 82 | 238S→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFFVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-49 | 83 | 238S→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFYVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-50 | 84 | 238S→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFWVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-51 | 85 | 82E→L | QNITEEFYQSTCSAVSKGYLSALRTGWYTSV ITIELSNIKENKCNGTDAKVKLIKQLLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-52 | 86 | 82E→I | QNITEEFYQSTCSAVSKGYLSALRTGWYTSV ITIELSNIKENKCNGTDAKVKLIKQILDKYKN AVTELQLLMQSTPATNNQARGSGSGRSLGFL LGVGSAIASGVAVSKVLHLEGEVNKIKSALL STNKAVVSLSNGVSVLTSKVLDLKNYIDKQL LPIVNKQSCSISNIETVIEFQQKNNRLLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-53 | 87 | 82E→M | QNITEEFYQSTCSAVSKGYLSALRTGWYTSV ITIELSNIKENKCNGTDAKVKLIKQMLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-54 | 88 | 82E→F | QNITEEFYQSTCSAVSKGYLSALRTGWYTSV ITIELSNIKENKCNGTDAKVKLIKQFLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-55 | 89 | 82E→Y | QNITEEFYQSTCSAVSKGYLSALRTGWYTSV ITIELSNIKENKCNGTDAKVKLIKQYLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-56 | 90 | 82E→W | QNITEEFYQSTCSAVSKGYLSALRTGWYTSV ITIELSNIKENKCNGTDAKVKLIKQWLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-57 | 91 | 86F→W | QNITEEFYQSTCSAVSKGYLSALRTGWYTSV ITIELSNIKENKCNGTDAKVKLIKQELDKWK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-58 | 92 | 89A→L | QNITEEFYQSTCSAVSKGYLSALRTGWYTSV ITIELSNIKENKCNGTDAKVKLIKQELDKYK NLVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-59 | 93 | 89A→I | QNITEEFYQSTCSAVSKGYLSALRTGWYTSV ITIELSNIKENKCNGTDAKVKLIKQELDKYK NIVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-60 | 94 | 89A→M | QNITEEFYQSTCSAVSKGYLSALRTGWYTSV ITIELSNIKENKCNGTDAKVKLIKQELDKYK NMVTELQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-61 | 95 | 89A→F | QNITEEFYQSTCSAVSKGYLSALRTGWYTSV ITIELSNIKENKCNGTDAKVKLIKQELDKYK NFVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-62 | 96 | 89A→Y | QNITEEFYQSTCSAVSKGYLSALRTGWYTSV ITIELSNIKENKCNGTDAKVKLIKQELDKYK NYVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-63 | 97 | 89A→W | QNITEEFYQSTCSAVSKGYLSALRTGWYTSV ITIELSNIKENKCNGTDAKVKLIKQELDKYK NWVTELQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-64 | 98 | 92E→L | QNITEEFYQSTCSAVSKGYLSALRTGWYTSV ITIELSNIKENKCNGTDAKVKLIKQELDKYK NAVTLLQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-65 | 99 | 92E→I | QNITEEFYQSTCSAVSKGYLSALRTGWYTSV ITIELSNIKENKCNGTDAKVKLIKQELDKYK NAVTILQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-66 | 100 | 92E→M | QNITEEFYQSTCSAVSKGYLSALRTGWYTSV ITIELSNIKENKCNGTDAKVKLIKQELDKYK NAVTMLQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-67 | 101 | 92E→F | QNITEEFYQSTCSAVSKGYLSALRTGWYTSV ITIELSNIKENKCNGTDAKVKLIKQELDKYK NAVTFLQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-68 | 102 | 92E→Y | QNITEEFYQSTCSAVSKGYLSALRTGWYTSV ITIELSNIKENKCNGTDAKVKLIKQELDKYK NAVTYLQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-69 | 103 | 92E→W | QNITEEFYQSTCSAVSKGYLSALRTGWYTSV ITIELSNIKENKCNGTDAKVKLIKQELDKYK NAVTWLQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-70 | 104 | 93L→M | QNITEEFYQSTCSAVSKGYLSALRTGWYTSV ITIELSNIKENKCNGTDAKVKLIKQELDKYK NAVTEMQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-71 | 105 | 93L→F | QNITEEFYQSTCSAVSKGYLSALRTGWYTSV ITIELSNIKENKCNGTDAKVKLIKQELDKYK NAVTEFQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-72 | 106 | 93L→Y | QNITEEFYQSTCSAVSKGYLSALRTGWYTSV ITIELSNIKENKCNGTDAKVKLIKQELDKYK NAVTEYQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-73 | 107 | 93L→W | QNITEEFYQSTCSAVSKGYLSALRTGWYTSV ITIELSNIKENKCNGTDAKVKLIKQELDKYK NAVTEWQLLMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-74 | 108 | 96L→M | QNITEEFYQSTCSAVSKGYLSALRTGWYTSV ITIELSNIKENKCNGTDAKVKLIKQELDKYK NAVTELQLMMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-75 | 109 | 96L→F | QNITEEFYQSTCSAVSKGYLSALRTGWYTSV ITIELSNIKENKCNGTDAKVKLIKQELDKYK NAVTELQLFMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-76 | 110 | 96L→Y | QNITEEFYQSTCSAVSKGYLSALRTGWYTSV ITIELSNIKENKCNGTDAKVKLIKQELDKYK NAVTELQLYMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-77 | 111 | 96L→W | QNITEEFYQSTCSAVSKGYLSALRTGWYTSV ITIELSNIKENKCNGTDAKVKLIKQELDKYK NAVTELQLWMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-78 | 112 | 193L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVMDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-79 | 113 | 193L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVFDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-80 | 114 | 193L→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVYDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-81 | 115 | 193L→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVWDLKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-82 | 116 | 195L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDMKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-83 | 117 | 195L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDFKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-84 | 118 | 195L→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDYKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-85 | 119 | 195L→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDWKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-86 | 120 | 199I→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYMDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-87 | 121 | 199I→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYFDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-88 | 122 | 199I→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYYDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-89 | 123 | 199I→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYWDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-90 | 124 | 190S→V | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTVKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-91 | 125 | 141L→M | QNITEEFYQSTCSAVSKGYLSALRTGWYTSV ITIELSNIKENKCNGTDAKVKLIKQELDKYK NAVTELQLLMQSTPATNNQARGSGSGRSLGF MLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-92 | 126 | 141L→F | QNITEEFYQSTCSAVSKGYLSALRTGWYTSV ITIELSNIKENKCNGTDAKVKLIKQELDKYK NAVTELQLLMQSTPATNNQARGSGSGRSLGF FLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-93 | 127 | 141L→Y | QNITEEFYQSTCSAVSKGYLSALRTGWYTSV ITIELSNIKENKCNGTDAKVKLIKQELDKYK NAVTELQLLMQSTPATNNQARGSGSGRSLGF YLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-94 | 128 | 141L→W | QNITEEFYQSTCSAVSKGYLSALRTGWYTSV ITIELSNIKENKCNGTDAKVKLIKQELDKYK NAVTELQLLMQSTPATNNQARGSGSGRSLGF WLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-95 | 129 | 373L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSMTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHS-96 | 130 | 373L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSFTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-97 | 131 | 373L→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSYTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHS-98 | 132 | 373L→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSWTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-1 | 133 | 227N→F, 230L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKFNRFLEITRE FSVNAGVTTPVSTYMLTNSELLSLINDMPITN DQKKLMSNNVQIVRQQSYSIMSIIKEEVLAY VVQLPLYGVIDTPCWKLHTSPLCTTNTKEGS NICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-2 | 134 | 227N→M, 82E→L | QNITEEFYQSTCSAVSKGYLSALRTGWY TSVITIELSNIKENKCNGTDAKVKLIKQLLDK YKNAVTELQLLMQSTPATNNQARGSGSGRS LGFLLGVGSAIASGVAVSKVLHLEGEVNKIK SALLSTNKAVVSLSNGVSVLTSKVLDLKNYI DKQLLPIVNKQSCSISNIETVIEFQQKMNRLL EITREFSVNAGVTTPVSTYMLTNSELLSLIND MPITNDQKKLMSNNVQIVRQQSYSIMSIIKEE VLAYVVQLPLYGVIDTPCWKLHTSPLCTTNT KEGSNICLTRTDRGWYCDNAGSVSFFPQAET CKVQSNRVFCDTMNSLTLPSEVNLCNVDIFN PKYDCKIMTSKTDVSSSVITSLGAIVSCYGKT KCTASNKNRGIIKTFSNGCDYVSNKGVDTVS VGNTLYYVNKQEGKSLYVKGEPIINFYDPLV FPSDEFDASISQVNEKINQSLAFIRKSDELL |
| FHM-3 | 135 | 227N→M, 89A→L | QNITEEFYQSTCSAVSKGYLSALRTGWYTSV ITIELSNIKENKCNGTDAKVKLIKQELDKYK NLVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-4 | 136 | 227N→M, 93L→F | QNITEEFYQSTCSAVSKGYLSALRTGWYTSV ITIELSNIKENKCNGTDAKVKLIKQELDKYK NAVTEFQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-5 | 137 | 227N→M, 96L→M | QNITEEFYQSTCSAVSKGYLSALRTGWYTSV ITIELSNIKENKCNGTDAKVKLIKQELDKYK NAVTELQLMMQSTPATNNQARGSGSGRSLG FLLGVGSAIASGVAVSKVLHLEGEVNKIKSA LLSTNKAVVSLSNGVSVLTSKVLDLKNYIDK QLLPIVNKQSCSISNIETVIEFQQKMNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-6 | 138 | 230L→F, 89A→F | QNITEEFYQSTCSAVSKGYLSALRTGWYTSV ITIELSNIKENKCNGTDAKVKLIKQELDKYK NFVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-7 | 139 | 230L→F, 82E→F | QNITEEFYQSTCSAVSKGYLSALRTGWYTSV ITIELSNIKENKCNGTDAKVKLIKQFLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-8 | 140 | 230L→F, 93L→F | QNITEEFYQSTCSAVSKGYLSALRTGWYTSV ITIELSNIKENKCNGTDAKVKLIKQELDKYK NAVTEFQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-9 | 141 | 230L→F, 238S→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRFLEITR EFMVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-10 | 142 | 227N→M, 234T→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRLLEIFR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-11 | 143 | 82E→F, 234T→F | QNITEEFYQSTCSAVSKGYLSALRTGWYTSV ITIELSNIKENKCNGTDAKVKLIKQFLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEIFR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-12 | 144 | 193L→M, 195L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVMDMKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-13 | 145 | 190S→V, 193L→M, 195L→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTVKVMDMKNYIDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-14 | 146 | 193L→M, 199I→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVMDLKNYMDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-15 | 147 | 193L→M, 199I→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVMDLKNYFDK QLLPIVNKQSCSISNIETVIEFQQKNNRLLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-16 | 148 | 141L→M, 373L→M | QNITEEFYQSTCSAVSKGYLSALRTGWYTSV ITIELSNIKENKCNGTDAKVKLIKQELDKYK NAVTELQLLMQSTPATNNQARGSGSGRSLGF MLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSMTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHM-17 | 149 | 141L→F, 373L→F | QNITEEFYQSTCSAVSKGYLSALRTGWYTSV ITIELSNIKENKCNGTDAKVKLIKQELDKYK NAVTELQLLMQSTPATNNQARGSGSGRSLGF FLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSFTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-18 | 150 | 82E→C, 224Q→C | QNITEEFYQSTCSAVSKGYLSALRTGWYTSV ITIELSNIKENKCNGTDAKVKLIKQCLDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFCQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-19 | 151 | 92E→C, 238S→C | QNITEEFYQSTCSAVSKGYLSALRTGWYTSV ITIELSNIKENKCNGTDAKVKLIKQELDKYK NAVTCLQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFCVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-20 | 152 | 79I→C, 220V→C | QNITEEFYQSTCSAVSKGYLSALRTGWYTSV ITIELSNIKENKCNGTDAKVKLCKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETCIEFQQKNNRLLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-21 | 153 | 97M→C, 291I→C | QNITEEFYQSTCSAVSKGYLSALRTGWYTSV ITIELSNIKENKCNGTDAKVKLIKQELDKYK NAVTELQLLCQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEITR EFSVNAGVTTPVSTYMLTNSELLSLINDMPIT NDQKKLMSNNVQIVRQQSYSIMSCIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-22 | 154 | 93L→C, 234T→C | QNITEEFYQSTCSAVSKGYLSALRTGWYTSV ITIELSNIKENKCNGTDAKVKLIKQELDKYK NAVTECQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEICR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-23 | 155 | 92E→C, 234T→C | QNITEEFYQSTCSAVSKGYLSALRTGWYTSV ITIELSNIKENKCNGTDAKVKLIKQELDKYK NAVTCLQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKNNRLLEICR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-24 | 156 | 227N→M, 230L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKSDELL |
| FHM-25 | 157 | 190S→V, 193L→M, 227N→M, 230L→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTVKVMDLKNYIDK QLLPIVNKQSCSISNIETVIEFQQKMNRFLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAFIRKSDELL |
| FHCS-1 | 158 | 227N→M, 230L→F, 509S→M | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKMDELL |
| FHCS-2 | 159 | 227N→M, 230L→F, 509S→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKFDELL |
| FHCS-3 | 160 | 227→M, 230L→F, 509S→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAFIRKLDELL |
| FHCS-4 | 161 | 227N→M, 230L→F, 505F→W | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAWIRKSDELL |
| FHCS-5 | 162 | 227N→M, 230L→F, 502S→Y | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLSEVNLCNVDIFNPKYD CKIMTSKTDVSSSVITSLGAIVSCYGKTKCTA SNKNRGIIKTFSNGCDYVSNKGVDTVSVGNT LYYVNKQEGKSLYVKGEPIINFYDPLVFPSDE FDASISQVNEKINQYLAFIRKSDELL |
| FHCM-1 | 163 | 227N→M, 230L→F, 505F→W, 509S→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINQSLAWIRKLDELL |
| FHCM-2 | 164 | 227N→M, 230L→F, 501Q→M, 509S→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTSKVLDLKNYIDKQ LLPIVNKQSCSISNIETVIEFQQKMNRFLEITR EFSVNAGVTTPVSTYMLTNSELLSLINDMPIT NDQKKLMSNNVQIVRQQSYSIMSIIKEEVLA YVVQLPLYGVIDTPCWKLHTSPLCTTNTKEG SNICLTRTDRGWYCDNAGSVSFFPQAETCKV QSNRVFCDTMNSLTLPSEVNLCNVDIFNPKY DCKIMTSKTDVSSSVITSLGAIVSCYGKTKCT ASNKNRGIIKTFSNGCDYVSNKGVDTVSVG NTLYYVNKQEGKSLYVKGEPIINFYDPLVFPS DEFDASISQVNEKINMSLAFIRKFDELL |
| FHCM-3 | 165 | 227N→M, 230L→F, 190S→V, 193L→M, 505F→W, 509S→L | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTVKVMDLKNYIDK QLLPIVNKQSCSISNIETVIEFQQKMNRFLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINQSLAWIRKLDELL |
| FHCM-4 | 166 | 227N→M, 230L→F, 190S→V, 193L→M, 501Q→M, 509S→F | QNITEEFYQSTCSAVSKGYLSALRTGWYTSV ITIELSNIKENKCNGTDAKVKLIKQELDKYK NAVTELQLLMQSTPATNNQARGSGSGRSLGF LLGVGSAIASGVAVSKVLHLEGEVNKIKSAL LSTNKAVVSLSNGVSVLTVKVMDLKNYIDK QLLPIVNKQSCSISNIETVIEFQQKMNRFLEIT REFSVNAGVTTPVSTYMLTNSELLSLINDMPI TNDQKKLMSNNVQIVRQQSYSIMSIIKEEVL AYVVQLPLYGVIDTPCWKLHTSPLCTTNTKE GSNICLTRTDRGWYCDNAGSVSFFPQAETCK VQSNRVFCDTMNSLTLPSEVNLCNVDIFNPK YDCKIMTSKTDVSSSVITSLGAIVSCYGKTK CTASNKNRGIIKTFSNGCDYVSNKGVDTVSV GNTLYYVNKQEGKSLYVKGEPIINFYDPLVF PSDEFDASISQVNEKINMSLAFIRKFDELL |

### DETAILED DESCRIPTION OF THE EMBODIMENTS

A mutant of a respiratory syncytial virus (RSV) fusion (F) protein is disclosed in the present disclosure. Those skilled in the art can refer to contents herein to appropriately improve process parameters for implementation. It is specifically noted that all similar substitutions and modifications will be apparent to those skilled in the art and are deemed to be included in the present disclosure. It will be apparent to those skilled in the art that modifications or suitable variations and combinations of what is described herein can be made to practice and apply the disclosed technology without departing from the content, spirit and scope of the present disclosure.

In the present disclosure, scientific and technical terms used herein have the meanings commonly understood by those skilled in the art, unless otherwise specified. Definitions of common terms in molecular biology can be found in Lewin's GENES XII, Jocelyn E.Krebs/Elliott S.Goldstein/Stephen T.Kilpatrick, by Jones & Bartlett Publishers, 2018.

Throughout the specification and claims, unless explicitly indicated otherwise, the term "comprise," or its variations of "include" or "contain," and the like, will be understood to include stated elements or components but not to exclude other elements or components. The terms "a," "an," and "the" include plural designators. The term "plurality" means two or more. The terms "such as," "for example," and the like are intended to refer to illustrative embodiments and are not intended to limit the scope of the present disclosure.

In order to enable those skilled in the art to better understand the technical solutions of the present disclosure, the present disclosure is described in further detail below in combination with specific examples.

### I. Definition

The term "wild-type," which may also be referred to as "natural," "native," refers to mutation that does not occur unnaturally. In some embodiments, illustrative instances of a wild-type RSV F protein are, for example, sequences shown in SEQ ID No. 1-6.

The term "mutation" refers to a change in a sequence (for example, nucleotide or amino acid sequence) relative to a corresponding sequence in a native, wild-type, standard, or reference form (that is, a non-mutated sequence). Amino acid mutation generally includes a substitution, a deletion or an insertion of an amino acid residue. The mutation can be artificial or naturally formed.

The terms "peptide," "polypeptide" and "protein" are used interchangeably and generally refer to peptides and proteins with amino acids covalently linked by peptide bonds. The term "protein" encompasses purified natural products, or products that may be produced partially or wholly by using recombinant or synthetic techniques. The terms "peptide" and "protein" may refer to aggregates of proteins, such as dimers or other multimers, fusion proteins, protein variants, or their derivatives. The terms also include proteins with modifications, for example, proteins modified by glycosylation, acetylation, phosphorylation, PEGylation, and ubiquitination. A protein may include amino acids that are not encoded by a codon of a nucleic acid. A protein may have an amino acid sequence with a sufficient length to produce of a tertiary and/or quaternary structure with higher levels.

The term "cleavage site for a protease" refers to a specific amino acid sequence motif within amino acids of a protein or a fusion protein. Within an amino acid sequence of a protein or a fusion protein, the protein or the fusion protein is cleaved by a specific protease that recognizes the amino acid sequence. In some embodiments, instances of the specific protease are, for example, furin, trypsin, factor Xa, thrombin, or cathepsin L.

The term "F0 precursor protein" refers to that an RSV F protein is first translated in vivo into a single chain inactive precursor protein F0 consisting of 574 amino acids. A signal peptide consisting of amino acids at positions 1 to 25 at an N-terminus of the F0 protein is removed by a signal peptidase. Furthermore, there are two furin cleavage sites at amino acids at positions 109/110 and 136/137 of the F0. Through the protease cleavage, a polypeptide with amino acids at positions 110 to 136 (referred to as p27) is removed, and an F1 consisting of amino acids at positions 137 to 574 and an F2 consisting of amino acids at positions 26 to 109 are generated.

The terms "head domain " and "tail domain" are defined in order to describe the structure of the RSV F protein visually in the present disclosure. The amino acids at positions 1 to 485 are defined as the head domain. The amino acid at position 486 to the C-terminus of the F protein are defined as the tail domain. The approximate spatial positions of the head domain and the tail domain are shown in FIG. 1A.

The term "virus-like particles (VLPs)" or "pseudoviruses" refers to a polyprotein structures composed of corresponding native viral structural proteins, but lacking the entire viral genome or part of the viral genome, particularly replicative and infectious components of the viral genome. Thus the virus-like particles are not replicative and infectious. The polyprotein structure closely mimics its corresponding native viral particles in morphology and size and can be spontaneously formed upon recombinant expression of the viral structural proteins.

The term "protein delivery vector" refers to a protein that can act as a carrier for an epitope peptide, that is, it can insert an epitope peptide into a specific position (for example, inside the protein, an N-terminal or a C-terminal), such that the epitope peptide can be presented and thus recognized by antibodies or an immune system.

The term "immunogenicity" refers to an ability of a substance to elicit, cause, stimulate or induce an immune response in an animal against a particular antigen, in the presence or absence of an adjuvant.

The term "conjugate" generally refers to any substance formed by joining together two or more independent moieties. For example, a conjugate may include a substance in which one polypeptide is linked to another polypeptide or polypeptides. When part of the conjugate is a protein, the conjugate is a protein conjugate.

### II. Mutation design of a head domain of an RSV F protein stabilizes the F protein in a prefusion trimer state

It has been reported that most of highly active neutralizing antibodies against the RSV F protein target a prefusion conformation (Ref: Battles M. B., McLellan J. S. Nat. Rev. Microbiol., 2019, 17:233-245; Gilman M. S. A. et al. PLoS Pathog., 2015, 11:e1005035; Harshbarger W., et al. MAbs, 2021, 13:1955812.), and a neutralizing antibody titer induced by an F protein trimer in a prefusion conformation is much higher than that induced by an F protein trimer in a postfusion conformation (Ref: Battles M. B., McLellan J. S. Nat. Rev. Microbiol., 2019, 17:233-245; Krarup A. et al. Nat. Commun., 2015, 6:8143), such that the F protein trimer in the prefusion conformation is a preferred antigen for development of recombinant subunit vaccines. However, the F protein trimer in the prefusion conformation is in a metastable state and is prone to a transition from the prefusion conformation (shown in FIG. 1A) to the postfusion conformation (shown in FIG. 1B). One of key issues in the development of the recombinant subunit vaccines is to stabilize the F protein in a prefusion trimer state through modification of a key site.

Spatial structures of a prefusion RSV F protein trimer and a postfusion RSV F protein trimer are obtained by X-ray crystal structure analysis (Ref: McLellan J. S., et al. Science, 2013, 340:1113-1117. McLellan J. S., et al. J. Virol., 2011, 85:7788-7796.), and submitted to a protein data bank (PDB). Spatial structure coordinates of the prefusion trimer and the postfusion trimer can be downloaded from the PDB according to PDB identifiers 4jhw and 3rrr. By using commonly used protein structure alignment software, such as Pymol and Chimera, the prefusion conformation and the postfusion conformation are subjected to structural superposition, as shown in FIG. 2. As can be seen from a structural alignment result of FIG. 2, from the prefusion conformation to the postfusion conformation, RR1 of the head domain and RR2 located at the C-terminal of the head domain and the tail domain undergo a large conformational change. In this case, α1 and α5 of the head domain also correspondingly undergo obvious conformational movement. Thus conformational changes of α1 and α5 are coupled with a conformational change of RR1. Based on the above structure analysis, stability mutation is introduced into an α1 or α5 region to prevent the conformational changes of α1 and α5. The conformational change of RR1 is prevented through coupling regulation. Thus the F protein can be stabilized in a prefusion conformation state. Furthermore, stability mutation can alternatively be introduced directly into an interaction region involved in RR1 to prevent the transition from the prefusion conformation to the postfusion conformation. For this reason, the present disclosure introduces mutation into an α1 helix (amino acids at positions 73 to 96) or an α5 helix (amino acids at positions 216 to 240) or directly into the interaction region involved in RR1, or any combination of the mutation. Interactions between residues are enhanced to stabilize conformations of α1, α5, or RR1, and thus the F protein is stabilized in the prefusion trimer state. Some illustrative single-site mutation design is shown in Table 4. The single-site mutation can be combined arbitrarily and can have similar or stronger stabilizing effects. Some illustrative mutation combinations are shown in Table 5.

**Table 4 Illustrative single-site mutation design for head domain of RSV F protein**

| **Mutant number** | **Introduced mutation** | **Mutant number** | **Introduced mutation** |
|---|---|---|---|
| FHS-1 | 227N→M | FHS-2 | 227N→G |
| FHS-3 | 227N→A | FHS-4 | 227N→V |
| FHS-5 | 227N→L | FHS-6 | 227N→I |
| FHS-7 | 227N→W | FHS-8 | 227N→F |
| FHS-9 | 227N→P | FHS-10 | 227N→S |
| FHS-11 | 227N→T | FHS-12 | 227N→C |
| FHS-13 | 227N→Y | FHS-14 | 227N→Q |
| FHS-15 | 227N→D | FHS-16 | 227N→E |
| FHS-17 | 227N→K | FHS-18 | 227N→R |
| FHS-19 | 227N→H | FHS-20 | 230L→M |
| FHS-21 | 230L→G | FHS-22 | 230L→A |
| FHS-23 | 230L→V | FHS-24 | 230L→I |
| FHS-25 | 230L→W | FHS-26 | 230L→F |
| FHS-27 | 230L→P | FHS-28 | 230L→S |
| FHS-29 | 230L→T | FHS-30 | 230L→C |
| FHS-31 | 230L→Y | FHS-32 | 230L→Q |
| FHS-33 | 230L→N | FHS-34 | 230L→D |
| FHS-35 | 230L→E | FHS-36 | 230L→K |
| FHS-37 | 230L→R | FHS-38 | 230L→H |
| FHS-39 | 234T→L | FHS-40 | 234T→I |
| FHS-41 | 234T→M | FHS-42 | 234T→F |
| FHS-43 | 234T→Y | FHS-44 | 234T→W |
| FHS-45 | 238S→L | FHS-46 | 238S→I |
| FHS-47 | 238S→M | FHS-48 | 238S→F |
| FHS-49 | 238S→Y | FHS-50 | 238S→W |
| FHS-51 | 82E→L | FHS-52 | 82E→I |
| FHS-53 | 82E→M | FHS-54 | 82E→F |
| FHS-55 | 82E→Y | FHS-56 | 82E→W |
| FHS-57 | 86F→W | FHS-58 | 89A→L |
| FHS-59 | 89A→I | FHS-60 | 89A→M |
| FHS-61 | 89A→F | FHS-62 | 89A→Y |
| FHS-63 | 89A→W | FHS-64 | 92E→L |
| FHS-65 | 92E→I | FHS-66 | 92E→M |
| FHS-67 | 92E→F | FHS-68 | 92E→Y |
| FHS-69 | 92E→W | FHS-70 | 93L→M |
| FHS-71 | 93L→F | FHS-72 | 93L→Y |
| FHS-73 | 93L→W | FHS-74 | 96L→M |
| FHS-75 | 96L→F | FHS-76 | 96L→Y |
| FHS-77 | 96L→W | FHS-78 | 193L→M |
| FHS-79 | 193L→F | FHS-80 | 193L→Y |
| FHS-81 | 193L→W | FHS-82 | 195L→M |
| FHS-83 | 195L→F | FHS-84 | 195L→Y |
| FHS-85 | 195L→W | FHS-86 | 199I→M |
| FHS-87 | 199I→F | FHS-88 | 199I→Y |
| FHS-89 | 199I→W | FHS-90 | 190S→V |
| FHS-91 | 141L→M | FHS-92 | 141L→F |
| FHS-93 | 141L→Y | FHS-94 | 141L→W |
| FHS-95 | 373L→M | FHS-96 | 373L→F |
| FHS-97 | 373L→Y | FHS-98 | 373L→W |

**Table 5 Illustrative site mutation combination design for head domain of RSV F protein**

| **Mutant number** | **Introduced mutation** |
|---|---|
| FHM-1 | 227N→F, 230L→F |
| FHM-2 | 227N→M, 82E→L |
| FHM-3 | 227N→M, 89A→L |
| FHM-4 | 227N→M, 93L→F |
| FHM-5 | 227N→M, 96L→M |
| FHM-6 | 230L→F, 89A→F |
| FHM-7 | 230L→F, 82E→F |
| FHM-8 | 230L→F, 93L→F |
| FHM-9 | 230L→F, 238S→M |
| FHM-10 | 227N→M, 234T→F |
| FHM-11 | 82E→F, 234T→F |
| FHM-12 | 193L→M, 195L→M |
| FHM-13 | 190S→V, 193L→M, 195L→M |
| FHM-14 | 193L→M, 199I→M |
| FHM-15 | 193L→M, 199I→F |
| FHM-16 | 141L→M, 373L→M |
| FHM-17 | 141L→F, 373L→F |
| FHM-18 | 82E→C, 224Q→C |
| FHM-19 | 92E→C, 238S→C |
| FHM-20 | 79I→C, 220V→C |
| FHM-21 | 97M→C, 291I→C |
| FHM-22 | 93L→C, 234T→C |
| FHM-23 | 92E→C, 234T→C |
| FHM-24 | 227N→M, 230L→F |
| FHM-25 | 227N→M, 230L→F, 190S→V, 193L→M |

### III. Example

Example 1 Plasmid construction, recombinant expression and effect validation of a mutant designed by introducing artificial mutation into a head domain (Table 4 and Table 5)

A natural RSV F protein is a homotrimer. In order to facilitate secretion expression of a protein in the example, amino acids at positions 514 to 574 including a transmembrane region and an intracellular region were removed from an F0 full-length sequence. Such a region plays an important role in formation of an F protein trimer. In order to assemble the cut sequence to form a trimer, a C-terminal of the cut sequence was linked to an exogenous trimerization domain (a T4 Foldon trimerization motif was used in the example) via a short linker (with a sequence being SAIG). Some studies showed that removing a sequence of p27 from an F0 contributed to trimer formation (Krarup A. et al. Nat. Commun., 2015, 6:8143.). In the example, a sequence (RARRELPRFMNYTLNNAKKTNVTLSKKRKRRF) at positions 107 to 138 containing p27 and a furin cleavage site was replaced by a linker QARGSGSGRS sequence. Moreover, a signal peptide MELLILKANAITTILTAVTFCFASG composed of amino acids at positions 1 to 25 at an N-terminal of the sequence was replaced by a signal peptide sequence MDMRVPAQLLGLLLLWLRGARC suitable for a recombinant expression system HEK293, so as to promote secretion expression of a recombinant protein. On this basis, corresponding mutants of the RSV F protein were constructed according to site mutation of the head domain designed in Table 4 and Table 5. Amino acid sequences of the constructed mutants (excluding the signal peptide, T4 foldon and a linker) were shown in SEQ ID No. 35-157.

According to the amino acid sequences shown in S EQ ID No. 35-157, the signal peptide suitable for the recombinant expression system HEK293, the T4 foldon trimerization motif, the linker, and an additional tag sequence useful for purification were added. Codon optimization was performed according to a codon bias of the HEK293 to form corresponding coding gene sequences. The coding gene sequences were constructed in a whole gene synthesis or point mutation mode. A Kozak sequence GCCACC was added at a front end of each sequence and then connected to a recombinant expression vector pcDNA3.1 to form a recombinant expression plasmid. An Expi293^{™} transient expression system (Thermo Fisher) was used to perform recombinant expression on the constructed mutants of the prefusion RSV F protein, and cell secretion supernatant was collected on the 5th day after transfection. As a control, a wild-type RSV F protein before artificial mutation (referred to as "wild-type RSV F protein 1 before artificial mutation") was subjected recombinant expression according to SEQ ID No. 12 based on the above method. Moreover, an RSV strain A2 F protein (Catalog Number: 11049-V08B) was purchased from Sino Biological, Inc. as another control (referred to as "wild-type RSV F protein 2 before artificial mutation"), whose amino acid sequence was shown in SEQ ID No.15.

In order to verify whether introduction of the above mutation can stabilize the RSV F protein in a prefusion trimer state, the example used a prefusion trimer-specifically binding monoclonal antibody AM14 to detect binding activity of the above constructed mutants with the AM14 antibody through an enzyme-linked immunosorbent assay (ELISA) experimental method. The binding activity of the constructed mutants was compared with AM14 monoclonal antibody binding activity of the wild-type RSV F protein 1 before artificial mutation and the wild-type RSV F protein 2 before artificial mutation. Published literature showed that the AM14 antibody binded to an epitope region at an interface between two adjacent monomers in the prefusion F protein trimer (Ref: Gilman M. S. A. et al. PLoS Pathog., 2015, 11:e1005035; Harshbarger W., et al. MAbs, 2021, 13:1955812.), and the epitope was disrupted when the F protein trimer transitions from the prefusion conformation to the postfusion conformation. Thus the AM14 antibody cannot bind to the postfusion F protein. The AM14 antibody was typically used for specific recognition and detection of a prefusion F protein trimer structure (Ref: Gilman M. S. A. et al. PLoS Pathog., 2015, 11:e1005035; Harshbarger W., et al. MAbs, 2021, 13:1955812.). Furthermore, it has been reported that the AM14 monoclonal antibody binding activity was positively correlated with a neutralizing antibody titer produced by immunized animals (Ref: Che Y. et al. Sci. Transl. Med., 2023, 15:eade6422.). Moreover, in order to verify whether the constructed mutants can be expressed, a His-tag antibody was used to detect expression of the mutants in the example.

An ELISA process of the transfection supernatant was as follows:

### (1) Assay of binding activity of AM14 monoclonal antibody

The AM14 monoclonal antibody (Wuhan Chemstan Biotechnology Co., Ltd.) was diluted to 1 µg/ml by using a coating buffer (Na₂CO₃-NaHCO₃ solution, pH9.6) and coated a 96-well ELISA plate at 100 µl/well at 4□ for 8 h to 12 h. The plate was washed by phosphate buffered saline with tween (PBST). A blocking buffer (20% calf serum, 0.2% yeast extract, the PBST was dissolved) was added at 100 µl/well and blocked at 37□ for 2 h. The plate was washed by the PBST. 10-fold diluted cell secretion supernatant of centrifuged recombinant expression mutants (including the wild-type RSV F protein 1 before artificial mutation) and 2 µg/ml wild-type RSV F protein 2 before artificial mutation were added at 100 µl/well and incubated at 37□ for 1 h. The plate was washed by the PBST. Diluted RSV F protein rabbit monoclonal antibodies labeled with horseradish peroxidase (1:1000, Sino Biological, Inc.) were added at 100 µl/well and incubated at 37□ for 1 h. The plate was washed by the PBST. Substrate solutions A and B were added in sequence for color development at room temperature for 5 min. A stop solution C was added to terminate chromogenic reaction. Optical density (OD) values were read at wavelengths of 450 nm and 630 nm.

### (2) Assay of His-tag antibody binding activity

Cell secretion supernatant of the centrifuged recombinant expression mutants (including the wild-type RSV F protein 1 before artificial mutation) was 10-fold diluted by using the coating buffer and then coated a 96-well ELISA plate. 2 µg/ml wild-type RSV F protein 2 before artificial mutation was used as a control well. Conditions were 100 µl/well at 4□ for 8 h to 12 h. The plate was washed by the PBST. The blocking buffer was added at 100 µl/well and blocked at 37□ for 2 h. The plate was washed by the PBST. Diluted His-tag antibodies (1:2000 dilution, Sino Biological, Inc.) were added at 100 µl/well and incubated at 37□ for 1 h. The plate was washed by the PBST. Diluted goat anti-mouse secondary antibodies labeled with horseradish peroxidase (1:10000, ZSBIO) were added at 100 µl/well and incubated at 37□ for 1 h. The plate was washed by the PBST. The substrate solutions A and B were added in sequence for color development at room temperature for 5 min. The stop solution C was added to terminate chromogenic reaction. Optical density (OD) values were read at wavelengths of 450 nm and 630 nm.

ELISA results are shown in Table 6, Table 7 and Table 8. The results show that His-tag antibody assay of most mutants has high OD values, indicating that most of the constructed mutants can be effectively expressed. Table 6 shows that the wild-type RSV F protein 1 before artificial mutation and the wild-type RSV F protein 2 before artificial mutation have no AM14 monoclonal antibody binding activity. Table 7 and Table 8 show that a plurality of single-site mutants at the head domain and a plurality of multi-site combination mutants at the head domain can bind to the AM14 monoclonal antibodies. The mutants include mutants FHS-1, FHS-4, FHS-5, FHS-6, FHS-11, FHS-12, FHS-14, FHS-26, FHS-45, FHS-46, FHS-47, FHS-48, FHS-49, FHS-50, FHS-65, FHS-66, FHS-67, FHS-68, FHS-69, FHS-76, FHS-78, FHS-84, FHS-87, FHS-88, FHM-1, FHM-2, FHM-5, FHM-9, FHM-12, FHM-13, FHM-15, FHM-16, FHM-18, FHM-19, FHM-20, FHM-22, FHM-23, FHM-24 and FHM-25. The OD values of the AM14 monoclonal antibody assay of these single-site mutants or multi-site combination mutants were significantly higher than those of the wild-type RSV F protein 1 before artificial mutation and the wild-type RSV F protein 2 before artificial mutation, indicating that the RSV F protein is successfully stabilized in the prefusion trimer state by introducing the above mutation.

**Table 6 Binding activity of wild-type RSV F protein 1 before artificial mutation and wild-type RSV F protein 2 before artificial mutation with AM14 monoclonal antibody and His-tag antibody**

| **Protein name** | **AM14 monoclonal antibody (OD value)** | **His-ta g antibody (OD value)** | **Protein name** | **AM14 monoclonal antibody (OD value)** | **His-tag antibody (OD value)** |
|---|---|---|---|---|---|
| Wild-type RSV F protein 1 before artificial mutation | 0.014 | 0.999 | Wild-type RSV F protein 2 before artificial mutation | 0.010 | 1.448 |

**Table 7 Binding activity of illustrative mutant of single-site mutation introduced into head domain of RSV F protein with AM14 monoclonal antibody and His-tag antibody**

| **Mutant number** | **AM14 monoclonal antibody (OD value)** | **His-tag antibody (OD value)** | **Mutant number** | **AM14 monoclonal antibody (OD value)** | **His-tag antibody (OD value)** |
|---|---|---|---|---|---|
| FHS-1 | 2.030 | 0.963 | FHS-50 | 2.296 | 1.013 |
| FHS-2 | 0.012 | 0.249 | FHS-51 | 0.006 | 0.353 |
| FHS-3 | 0.076 | 0.554 | FHS-52 | 0.007 | 0.291 |
| FHS-4 | 2.256 | 1.452 | FHS-53 | 0.008 | 0.348 |
| FHS-5 | 2.239 | 1.640 | FHS-54 | 0.012 | 0.111 |
| FHS-6 | 2.646 | 1.253 | FHS-55 | 0.009 | 2.217 |
| FHS-7 | 0.011 | 0.364 | FHS-56 | 0.009 | 0.117 |
| FHS-8 | 0.013 | 0.343 | FHS-57 | 0.007 | 0.118 |
| FHS-9 | 0.009 | 0.079 | FHS-58 | 0.011 | 0.372 |
| FHS-10 | 0.069 | 0.228 | FHS-59 | 0.006 | 0.804 |
| FHS-11 | 2.043 | 1.009 | FHS-60 | 0.007 | 0.229 |
| FHS-12 | 1.238 | 1.208 | FHS-61 | 0.008 | 0.721 |
| FHS-13 | 0.013 | 0.161 | FHS-62 | 0.010 | 0.136 |
| FHS-14 | 2.612 | 0.968 | FHS-63 | 0.006 | 0.139 |
| FHS-15 | 0.091 | 0.351 | FHS-64 | 0.008 | 1.194 |
| FHS-16 | 0.009 | 0.112 | FHS-65 | 1.732 | 0.789 |
| FHS-17 | 0.009 | 0.137 | FHS-66 | 1.750 | 1.093 |
| FHS-18 | 0.009 | 0.109 | FHS-67 | 1.859 | 0.537 |
| FHS-19 | 0.007 | 0.187 | FHS-68 | 1.989 | 0.937 |
| FHS-20 | 0.009 | 0.195 | FHS-69 | 1.875 | 0.750 |
| FHS-21 | 0.008 | 0.121 | FHS-70 | 0.007 | 0.233 |
| FHS-22 | 0.010 | 0.115 | FHS-71 | 0.012 | 0.489 |
| FHS-23 | 0.008 | 0.326 | FHS-72 | 0.007 | 0.132 |
| FHS-24 | 0.010 | 0.503 | FHS-73 | 0.016 | 0.181 |
| FHS-25 | 0.007 | 0.109 | FHS-74 | 0.023 | 1.149 |
| FHS-26 | 0.841 | 1.751 | FHS-75 | 0.028 | 0.778 |
| FHS-27 | 0.005 | 0.097 | FHS-76 | 0.821 | 0.322 |
| FHS-28 | 0.008 | 0.107 | FHS-77 | 0.060 | 0.687 |
| FHS-29 | 0.007 | 0.099 | FHS-78 | 1.235 | 1.200 |
| FHS-30 | 0.009 | 0.095 | FHS-79 | 0.008 | 0.500 |
| FHS-31 | 0.007 | 0.102 | FHS-80 | 0.005 | 0.504 |
| FHS-32 | 0.008 | 0.099 | FHS-81 | 0.008 | 0.349 |
| FHS-33 | 0.005 | 0.071 | FHS-82 | 0.063 | 0.958 |
| FHS-34 | 0.007 | 0.103 | FHS-83 | 0.064 | 1.740 |
| FHS-35 | 0.007 | 0.103 | FHS-84 | 1.006 | 1.549 |
| FHS-36 | 0.009 | 0.123 | FHS-85 | 0.008 | 0.217 |
| FHS-37 | 0.007 | 0.111 | FHS-86 | 0.062 | 1.172 |
| FHS-38 | 0.008 | 0.097 | FHS-87 | 0.239 | 1.536 |
| FHS-39 | 0.007 | 0.365 | FHS-88 | 2.296 | 0.623 |
| FHS-40 | 0.009 | 0.571 | FHS-89 | 0.006 | 0.675 |
| FHS-41 | 0.006 | 0.368 | FHS-90 | 0.007 | 2.138 |
| FHS-42 | 0.010 | 0.185 | FHS-91 | 0.008 | 0.810 |
| FHS-43 | 0.006 | 0.349 | FHS-92 | 0.012 | 1.159 |
| FHS-44 | 0.039 | 0.494 | FHS-93 | 0.009 | 0.422 |
| FHS-45 | 0.120 | 1.135 | FHS-94 | 0.009 | 0.714 |
| FHS-46 | 2.410 | 1.630 | FHS-95 | 0.007 | 1.721 |
| FHS-47 | 1.317 | 1.462 | FHS-96 | 0.011 | 0.866 |
| FHS-48 | 2.422 | 0.732 | FHS-97 | 0.006 | 0.878 |
| FHS-49 | 1.957 | 0.721 | FHS-98 | 0.007 | 0.288 |

**Table 8 Binding activity of illustrative mutant of multi-site combination mutation introduced into head domain of RSV F protein with AM14 monoclonal antibody and His-tag antibody**

| **Mutant number** | **AM14 monoclonal antibody (OD value)** | **His-tag antibody (OD value)** |
|---|---|---|
| FHM-1 | 2.288 | 0.489 |
| FHM-2 | 2.228 | 1.585 |
| FHM-3 | 0.070 | 0.359 |
| FHM-4 | 0.028 | 0.183 |
| FHM-5 | 1.964 | 0.982 |
| FHM-6 | 0.005 | 0.098 |
| FHM-7 | 0.007 | 0.196 |
| FHM-8 | 0.016 | 0.465 |
| FHM-9 | 2.121 | 1.446 |
| FHM-10 | 0.010 | 0.136 |
| FHM-11 | 0.006 | 0.139 |
| FHM-12 | 2.128 | 1.708 |
| FHM-13 | 1.070 | 0.443 |
| FHM-14 | 0.008 | 0.458 |
| FHM-15 | 1.505 | 1.720 |
| FHM-16 | 0.260 | 1.280 |
| FHM-17 | 0.006 | 0.654 |
| FHM-18 | 1.797 | 0.870 |
| FHM-19 | 0.367 | 0.228 |
| FHM-20 | 0.607 | 0.586 |
| FHM-21 | 0.007 | 0.159 |
| FHM-22 | 1.978 | 1.765 |
| FHM-23 | 1.887 | 1.620 |
| FHM-24 | 1.610 | 0.932 |
| FHM-25 | 2.419 | 1.728 |

### Example 2 Purification, identification and biological activity evaluation of illustrative mutants FHM-24 and FHM-25 with mutation introduced into the head domain expressed in Example 1

Recombinant expression plasmids of constructed illustrative mutants FHM-24 and FHM-25 (corresponding amino acid sequences SEQ ID No. 156 and SEQ ID No. 157) with the mutation introduced into the head domain of the RSV F protein were transiently expressed by the Expi293^{™} expression system (Thermo Fisher). Secretion supernatant was collected on the 5th day after transfection. Affinity chromatography purification was performed on the transfection supernatant by using a nickel column (HisTrap FF crude pre-packed column). An equilibration buffer (20 mM phosphate-150 mM sodium chloride, pH 7.4) was used for equilibrating the chromatographic column. A sample was loaded. A target protein was collected after elution in 20% and 50% elution buffers (20 mM phosphate-150 mM sodium chloride-500 mM imidazole, pH 7.4). The purified protein was subjected to 12% SDS-PAGE analysis. Results were shown in FIG. 3. A molecular weight corresponding to a target protein monomer band was about 50 kD to 70 kD, which was basically consistent with a theoretical value, indicating that the mutants FHM-24 and FHM-25 can be expressed correctly.

After purified samples of the mutants FHM-24 and FHM-25 were obtained, the binding activity of the two mutants with the AM14 monoclonal antibody was detected by antibody binding experiments by using prefusion trimer specific-monoclonal antibody AM14 to verify whether the mutants were successfully stabilized in prefusion trimer state. Moreover, folding of the mutants FHM-24 and FHM-25 was further verified by using Synagis^{®}. Synagis^{®} can bind specifically to epitope II of the RSV F protein. The prefusion F protein and the postfusion F protein contain epitope II in a same conformation. Thus Synagis^{®} can bind to the prefusion F protein and the postfusion F protein. In the experiment, the wild-type RSV F protein 1 before artificial mutation and the wild-type RSV F protein 2 before artificial mutation were used as controls. A specific operation process of the experiment is as follows: the purified mutants FHM-24 and FHM-25, the wild-type RSV F protein 1 before artificial mutation, and the wild-type RSV F protein 2 before artificial mutation were subjected to 2-fold serial dilutions from initial concentrations of 80 µg/ml (corresponding primary antibody was AM14 monoclonal antibody) and 10 µg/ml (corresponding primary antibody was Synagis^{®}) by using a coating buffer and coated an ELISA plate at 100 µl/well. 3 duplicate wells were made for each dilution. Coating was performed for 8 h to 12 h at 4□. Blank wells were use as a negative control. The plate was washed by a PBST. A blocking buffer was added and blocked at 37□ for 2 h. The plate was washed by the PBST. Diluted AM14 monoclonal antibodies (0.1 µg/ml) or Synagis^{®} (2 ug/ml) was added at 100 µl/well and incubated at 370 for 1 h. The plate was washed by the PBST. Diluted goat anti-human secondary antibody labeled with horseradish peroxidase (1:10000) was added at 100 µl/well and incubated at 37□ for 1 h. The plate was washed by the PBST. Substrate solutions A and B were added in sequence for color development at room temperature for 5 min. A stop solution C was added to terminate chromogenic reaction. OD values were read at wavelengths of 450 nm and 630 nm. Mean OD_{450/630nm} values of the duplicate wells with different concentrations were calculated, and a standard deviation was calculated. With protein concentrations as an abscissa and OD_{450/630nm} mean values corresponding to concentrations as an ordinate, a broken line graph was drawn by Origin software.

Results of antibody binding experiments were shown in FIG. 4 and FIG. 5. The results of the AM14 monoclonal antibody binding experiment (FIG. 4) show that the wild-type RSV F protein 1 before artificial mutation and the wild-type RSV F protein 2 before artificial mutation have no AM14 monoclonal antibody binding activity, and the illustrative mutants FHM-24 and FHM-25 with the mutation introduced into the head domain exhibit AM14 monoclonal antibody binding activity. The results indicate that the RSV F protein can be stabilized in the prefusion trimer state by introducing the stabilizing mutation into the head domain, to prevent the transition conformational from a prefusion conformation to a postfusion conformation. The results of the Synagis^{®} binding experiment (FIG. 5) show that the mutants FHM-24 and FHM-25, the wild-type RSV F protein 1 before artificial mutation and the wild-type RSV F protein 2 before artificial mutation all have Synagis^{®} binding activity. All the four proteins can fold correctly.

### Example 3: On the basis of introducing mutation into a head domain of an RSV F protein, mutation design of a tail domain is added to further enhance stability of a prefusion RSV F protein trimer, and further to improve immunogenicity

Stability of a prefusion RSV F protein trimer structure has an important influence on the immunogenicity of the trimer as an antigen. On the basis of the mutation introduced into the head domain in the above examples, mutation was also introduced in a tail domain of the F protein in the example. By enhancing an interaction between triple helix bundles in RR2, the stability of the F protein trimer structure was improved, and AM14 monoclonal antibody binding activity was enhanced, so as to further improve the immunogenicity. It was reported that the AM14 monoclonal antibody binding activity positively correlated with a neutralizing antibody titer produced by immunized animals (Ref: Che Y. et al. Sci. Transl. Med., 2023, 15:eade6422.). Thus, in the example, single-site mutation or a multi-site mutation combination was introduced into RR2 of the tail domain of the RSV F protein to further enhance the stability of the prefusion F protein trimer structure by enhancing the interaction between the triple helix bundles in RR2. In this example, mutants FHM-24 (mutation of 227N → M and 230L → F was introduced in the head domain) and FHM-25 (mutation of 227N → M, 230L → F, 190S → V and 193L → M was introduced into the head domain) of mutation introduced into the head domain were taken as instances to further enhance the stability of the RSV F protein trimer by further introducing the stability mutation into the tail domain. Some illustrative single-site mutation design of the tail domain is shown in Table 9. The single-site mutation of the tail domain can be combined arbitrarily and can have similar or stronger stabilizing effects. Some illustrative mutation combinations are shown in Table 10 and Table 11.

**Table 9 Illustrative single-site mutation design introduced into the tail domain based on the mutant FHM-24, to further enhance the stability of the prefusion RSV F protein trimer**

| **Mutant number** | **Mutation introduced into the head domain** | **Single-site mutation introduced into the tail domain** |
|---|---|---|
| FHCS-1 | 227N→M, 230L→F | 509S→M |
| FHCS-2 | 227N→M, 230L→F | 509S→F |
| FHCS-3 | 227N→M, 230L→F | 509S→L |
| FHCS-4 | 227N→M, 230L→F | 505F→W |
| FHCS-5 | 227N→M, 230L→F | 502S→Y |

**Table 10 Illustrative site mutation combination design introduced into the tail domain based on the mutant FHM-24, to further enhance the stability of the prefusion RSV F protein trimer**

| **Mutant number** | **Mutation introduced into the head domain** | **Site mutation combination introduced into the tail domain** |
|---|---|---|
| FHCM-1 | 227N→M, 230L→F | 505F→W,509S→L |
| FHCM-2 | 227N→M, 230L→F | 501Q→M,509S→F |

**Table 11 Illustrative site mutation design introduced into the tail domain based on the mutant FHM-25, to further enhance the stability of the prefusion RSV F protein trimer**

| **Mutant number** | **Mutation introduced into the head domain** | **Site mutation combination introduced into the tail domain** |
|---|---|---|
| FHCM-3 | 227N→M, 230L→F, 190S→V, 193L→M | 505F→W,509S→L |
| FHCM-4 | 227N→M, 230L→F, 190S→V, 193L→M | 501Q→M,509S→F |

### Example 4 Plasmid construction, recombinant expression, purification and biological activity validation of the mutant designed in Example 3

In order to verify whether the mutation introduced into the tail domain can further enhance the stability of the prefusion RSV F protein trimer, the example used a prefusion F protein trimer-specifically binding monoclonal antibody AM14 to assay antibody binding activity of the mutant designed in Example 3. The antibody binding activity was compared with AM14 antibody binding activity of the mutants FHM-24 or FHM-25 without mutation introduced into the tail domain, the wild-type RSV F protein 1 before artificial mutation and the wild-type RSV F protein 2 before artificial mutation to evaluate an enhancement effect of the mutation introduced into the tail domain on the stability of the prefusion RSV F protein trimer.

First, amino acid sequences corresponding to the mutants of the RSV F protein designed in Example 3 were SEQ ID No. 158-166 in sequence. According to the corresponding amino acid sequences, the signal peptide suitable for the recombinant expression system HEK293, the T4 foldon trimerization motif, the linker, and an additional tag sequence useful for purification were added. Codon optimization was performed according to a codon bias of the HEK293 to form corresponding coding gene sequences. Recombinant expression plasmids were constructed by using the plasmid construction and recombinant expression processes described in Example 1. Recombinant expression of the mutants was performed by using an Expi293^{™}transient expression system. Cell secretion supernatant was collected on the 5th day after transfection.

Furthermore, all mutants designed in Example 3 were purified through a purification method described in Example 1, to obtain a purified mutant protein. The purified protein was subjected to 12% SDS-PAGE analysis. Results were shown in FIG. 6. A molecular weight corresponding to a target protein monomer band was about 50 kD to 70 kD, indicating that the mutants can be expressed correctly.

Finally, AM14 monoclonal antibody binding activity was assayed for a purified mutant sample obtained by purification through the ELISA described in Example 2 by using a prefusion RSV F protein trimer-specifically binding monoclonal antibody AM14. The AM14 monoclonal antibody binding activity was compared with AM14 monoclonal antibody binding activity of the mutants FHM-24 or FHM-25 without mutation introduced into the tail domain, the wild-type RSV F protein 1 before artificial mutation, and the wild-type RSV F protein 2 before artificial mutation to evaluate the enhancement effect of the mutation introduced into the tail domain on the stability of the prefusion RSV F protein trimer. Moreover, folding of the constructed mutants was further verified by using Synagis^{®}.

Results of an AM14 monoclonal antibody binding experiment were shown in FIG. 7A and FIG. 7B. The results show that the wild-type RSV F protein 1 before artificial mutation and the wild-type RSV F protein 2 before artificial mutation have no AM14 monoclonal antibody binding activity. The F protein mutants with mutation introduced into the head domain and the tail domain have significantly increased AM14 monoclonal antibody binding activity compared with the mutants (FHM-24, FHM-25) without mutation introduced into the tail domain, the wild-type RSV F protein 1 before artificial mutation, and the wild-type RSV F protein 2 before artificial mutation. The results indicate that the mutation introduced into the tail domain can further enhance the stability of the prefusion RSV F protein trimer structure. Results of a Synagis^{®} binding experiment were shown in FIG. 8A and FIG. 8B. The results show that the wild-type RSV F protein 1 before artificial mutation, the wild-type RSV F protein 2 before artificial mutation, the F protein mutants without mutation introduced into the tail domain, and the mutants with stability mutation introduced into the tail domain have strong Synagis^{®} binding activity, indicating that these proteins can fold correctly.

### Example 5 An accelerated stability test study of illustrative constructed mutants of an RSV F protein

The stability of the constructed mutants of the RSV F protein is an important characteristic to be paid attention when the mutants are used in vaccine development as antigens. In the example, a thermal accelerated stability test study was carried out on constructed illustrative mutants FHCS-1, FHCS-2 and FHCS-4 of the RSV F protein. Mutant samples obtained after expression purification were stored at 37°C for 2 weeks and 4 weeks respectively. Meanwhile, same samples were stored at -80°C for 2 weeks and 4 weeks as controls. Stability samples were subjected to 2-fold serial dilutions from initial concentrations of 10 µg/ml (corresponding primary antibody was AM14 monoclonal antibody) and 2.5 µg/ml (corresponding primary antibody was Synagis^{®}) by using a coating buffer and coated an ELISA plate at 100 µl/well. 2 duplicate wells were prepared for each dilution. AM14 monoclonal antibody binding activity and Synagis^{®} binding activity of the samples were assayed through the ELISA method described in Example 2.

The results of a thermal accelerated stability test were shown in Table 12, Table 13, Table 14, and Table 15. The results show that after the mutants of the RSV F protein constructed in the present disclosure are placed at 37□ for 4 weeks, the AM14 monoclonal antibody binding activity and the Synagis^{®} binding activity do not change obviously compared with the samples placed at -80□, indicating that prefusion trimer structures of the mutants of the RSV F protein constructed in the present disclosure are still stable after being placed at 37□ for 4 weeks.

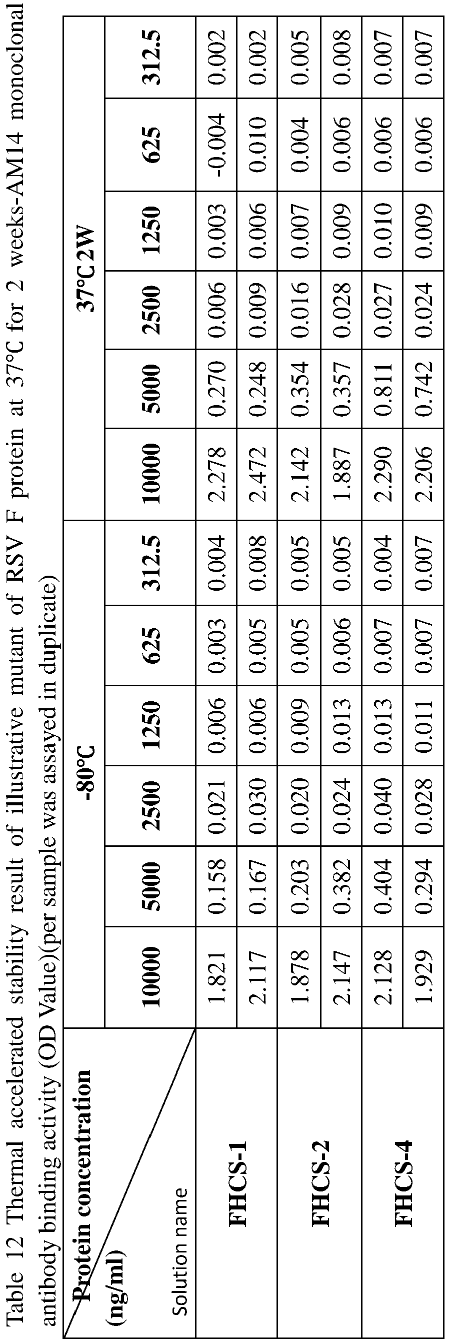

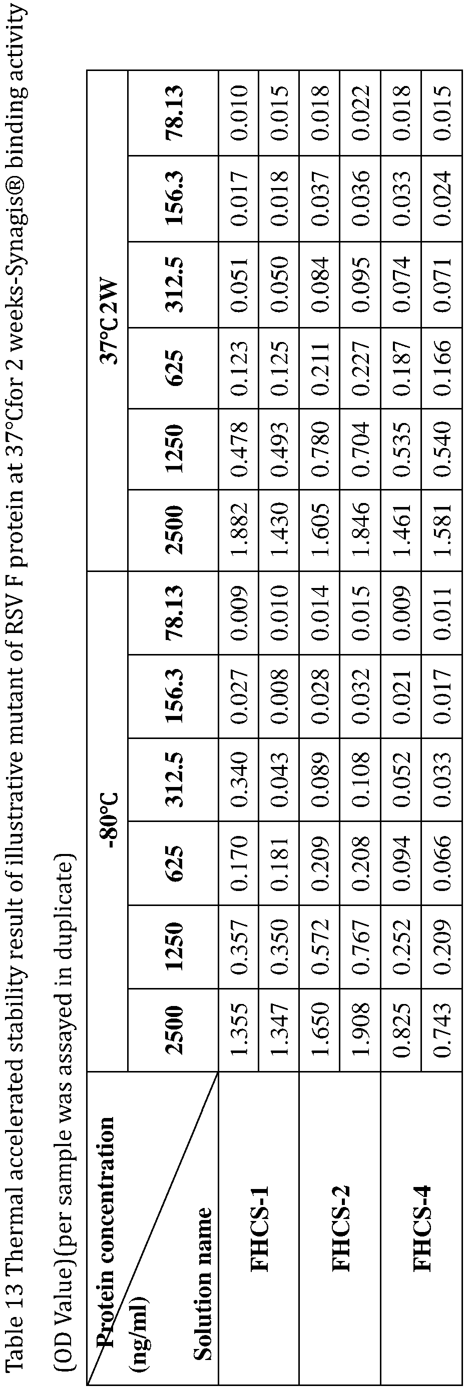

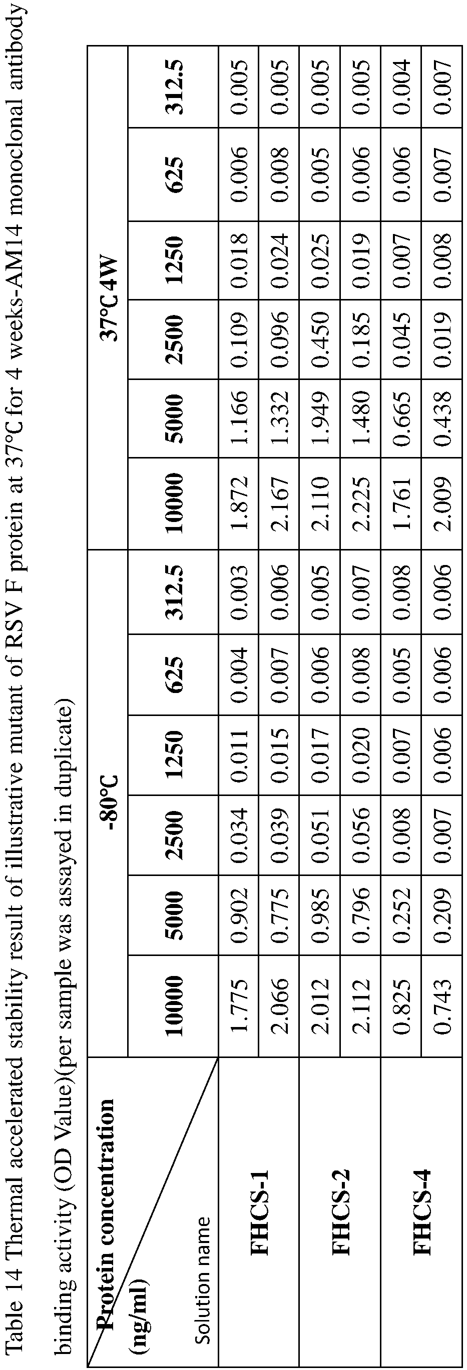

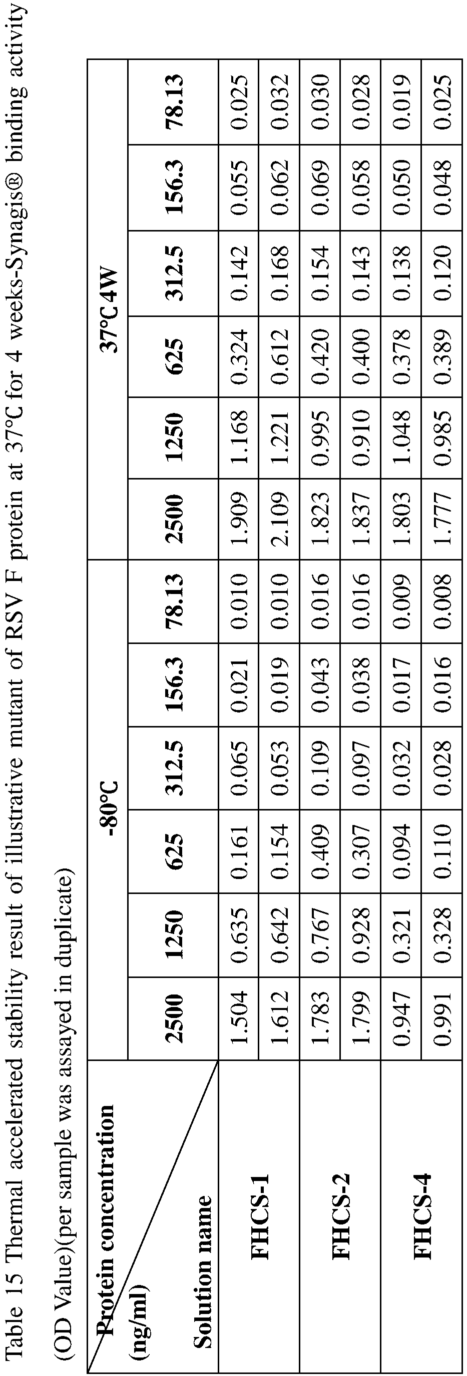

### Example 6 Evaluation of animal immunization effects of illustrative constructed mutants of an RSV F protein

In order to verify whether the immunization effects of the mutants of the RSV F protein constructed in the present disclosure is significantly enhanced, animal immunization effect evaluation studies were carried out on mutants FHCS-1, FHCS-2, FHCS-3 and FHCS-4 in the example. The immunization effects were compared with immunization effects of a wild-type RSV F protein before artificial mutation and a mutant protein FHM-24 with mutation only introduced into a head domain.

A specific experimental method was as follows: a purified illustrative mutant of the F protein and the wild-type RSV F protein 2 before artificial mutation were mixed with an aluminum hydroxide adjuvant separately, and then a polyinosinic solution was added to prepare vaccines. BALB/c mice (purchased from Beijing Vital River Laboratory Animal Technology Co., Ltd. , SPF grade, female, 6 weeks to 8 weeks old) were immunized with the prepared vaccines, and each group had 10 mice. The mice were immunized intramuscularly at week 0 and week 2 with 0.5 µg/mouse antigen and 0.1 ml/mouse immune volume. Blood samples were collected 1 week after the last immunization. A live RSV strain A2 neutralization experiment was used to assay a neutralizing antibody level in immune serum. Specifically, a maintenance medium (DMEM medium containing 2% fetal bovine serum and 1% streptomycin) was used for 2-fold serial dilutions on the serum. Diluted RSV A2 viruses were added at 100 TCID₅₀/well. Duplicate wells were made for each piece of serum. A virus control well without serum and a cell control well without serum and viruses were set. Incubation was performed at 4□ for 2 h. A Hep2 cell suspension was added at 2*104 cells per well and cultured in 37□, in a 5% CO2 incubator for 3 days. Neutralization results were determined through an immunofluorescence method. A serum dilution multiple lower than 50% of a mean value of fluorescent spots in the virus control well was taken as a neutralizing antibody titer of the serum.

Results were shown in FIG. 9, compared with the wild-type RSV F protein before artificial mutation, the mutant had an increased neutralizing antibody titer after mutation was introduced into the head domain. A geometric mean titer (GMT) of neutralizing antibodies increased from 147 to 239. Furthermore, after the mutation was introduced into the head domain and the tail domain, the neutralizing antibody titer was further increased significantly. The GMT increased to 588 to 1261, which was 4 times to 8.58 times higher than that of the wild-type RSV F protein before artificial mutation. The results show that the RSV F protein is stabilized in the prefusion trimer structure state by introducing the mutation into the head domain and the tail domain. Immunogenicity of the protein is obviously improved. Candidate antigens with obvious technical advantages and immunoactivity advantages are provided for development of recombinant RSV vaccines.

What are described above are merely preferred embodiments of the present disclosure. It should be pointed out that those of ordinary skill in the art can make several improvements and modifications without departing from the principles of the present disclosure, and these improvements and modifications should also be deemed as falling within the scope of protection of the present disclosure.

## Claims

1. A mutant of a wild-type respiratory syncytial virus (RSV) fusion (F) protein, being an artificial mutant, wherein compared with a wild-type RSV F protein before artificial mutation, the mutant has at least one amino acid mutation site, and the amino acid mutation site comprises:
(a) any one of amino acids at positions 73 to 96; or
(b) any one of amino acids at positions 216 to 240; or
(c) an amino acid at position 141, an amino acid at position 190, an amino acid at position 193, an amino acid at position 195, an amino acid at position 199, or an amino acid at position 373.

2. The mutant according to claim 1, **characterized in that** compared with the wild-type RSV F protein before artificial mutation, the mutant has a plurality of amino acid mutation sites, and the plurality of amino acid mutation sites comprise:
(a) at least two sties selected from the amino acids at positions 73 to 96; or
(b) at least two sties selected from the amino acids at positions 216 to 240; or
(c) at least one site selected from the amino acids at positions 73 to 96, and at least one site selected from the amino acids at positions 216 to 240.

3. The mutant according to claim 2, **characterized in that** compared with the wild-type RSV F protein before artificial mutation, the mutant has the plurality of amino acid mutation sites, and the plurality of amino acid mutation sites comprise a combination of any one of the following (a), (b) or (c) with (d):
(a) at least two sties selected from the amino acids at positions 73 to 96;
(b) at least two sties selected from the amino acids at positions 216 to 240;
(c) at least one site selected from the amino acids at positions 73 to 96, and at least one site selected from the amino acids at positions 216 to 240; and
(d) at least one site selected from the amino acid at position 141, the amino acid at position 190, the amino acid at position 193, the amino acid at position 195, the amino acid at position 199, or the amino acid at position 373.

4. The mutant according to claim 1, 2 or 3, **characterized in that** the amino acid mutation sites further comprise at least one site selected from amino acids at positions 486 to 513.

5. The mutant according to claim 1, **characterized in that** the wild-type RSV F protein is subtype A or subtype B.

6. The mutant according to claim 5, **characterized in that** an amino acid sequence of the wild-type RSV F protein is a sequence shown in SEQ ID No. 1-6, or a sequence having identity greater than 90% and having same or substantially same biological functions as the sequence shown in SEQ ID No. 1-6.

7. The mutant according to claim 1, **characterized in that** the wild-type RSV F protein before artificial mutation is in the following form:
(a) an F0 precursor protein comprising two protease cleavage sites; or
(b) an F0 precursor protein that is incapable of binding to a protease; or
(c) a combination of an independent F1 polypeptide and an independent F2 polypeptide; or
(d) a combination of an F2 polypeptide, a linker, and an F1 polypeptide; or
(e) a combination of an F2' polypeptide, a linker, and an F1' polypeptide, wherein the F2' polypeptide is 1 to 10 amino acid residues less than the F2 polypeptide in an N-terminal direction, and the F1' polypeptide is 1 to 10 amino acid residues less than the F1 polypeptide in a C-terminal direction.

8. The mutant according to claim 7, **characterized in that** the protease is furin.

9. The mutant according to claim 7, **characterized in that** an amino acid sequence of the wild-type RSV F protein before artificial mutation is a sequence shown in SEQ ID No. 7-34, or a sequence having identity greater than 90% and having same or substantially same biological functions as the sequence shown in SEQ ID No. 7-34.

10. The mutant according to claim 1, **characterized in that** the amino acid mutation site comprises at least one site selected from the amino acid at position 79, the amino acid at position 82, the amino acid at position 92, the amino acid at position 93, the amino acid at position 96, the amino acid at position 141, the amino acid at position 190, the amino acid at position 193, the amino acid at position 195, the amino acid at position 199, the amino acid at position 220, the amino acid at position 224, the amino acid at position 227, the amino acid at position 230, the amino acid at position 234, the amino acid at position 238, or the amino acid at position 373 .

11. The mutant according to claim 4, **characterized in that** the amino acid mutation site further comprises at least one site selected from the amino acid at position 501, the amino acid at position 502, the amino acid at position 505, the amino acid at position 509, or the amino acid at position 512.

12. The mutant according to claim 10, **characterized in that** the amino acid mutation site comprises the following:
(1) the amino acid at position 92; or
(2) the amino acid at position 96; or
(3) the amino acid at position 227; or
(4) the amino acid at position 230; or
(5) the amino acid at position 238; or
(6) the amino acid at position 227 and the amino acid at position 230; or
(7) the amino acid at position 82 and the amino acid at position 227; or
(8) the amino acid at position 89 and the amino acid at position 227; or
(9) the amino acid at position 92 and the amino acid at position 227; or
(10) the amino acid at position 92 and the amino acid at position 230; or
(11) the amino acid at position 96 and the amino acid at position 227; or
(12) the amino acid at position 96 and the amino acid at position 230; or
(13) the amino acid at position 96 and the amino acid at position 238; or
(14) the amino acid at position 93 and the amino acid at position 227; or
(15) the amino acid at position 89 and the amino acid at position 230; or
(16) the amino acid at position 82 and the amino acid at position 230; or
(17) the amino acid at position 93 and the amino acid at position 230; or
(18) the amino acid at position 227 and the amino acid at position 238; or
(19) the amino acid at position 230 and the amino acid at position 238; or
(20) the amino acid at position 227 and the amino acid at position 234; or
(21) the amino acid at position 82 and the amino acid at position 234; or
(22) the amino acid at position 193 and the amino acid at position 195; or
(23) the amino acid at position 190, the amino acid at position 193, and the amino acid at position 195; or
(24) the amino acid at position 193 and the amino acid at position 199; or
(25) the amino acid at position 141 and the amino acid at position 373; or
(26) the amino acid at position 82 and the amino acid at position 224; or
(27) the amino acid at position 92 and the amino acid at position 238; or
(28) the amino acid at position 79 and the amino acid at position 220; or
(29) an amino acid at position 97 and an amino acid at position 291; or
(30) the amino acid at position 93 and the amino acid at position 234; or
(31) the amino acid at position 92 and the amino acid at position 234; or
(32) the amino acid at position 193, the amino acid at position 227, and the amino acid at position 230; or
(33) the amino acid at position 190, the amino acid at position 193, and the amino acid at position 230; or
(34) the amino acid at position 193 and the amino acid at position 230; or
(35) the amino acid at position 190, the amino acid at position 193, the amino acid at position 227, and the amino acid at position 230; or
(36) the amino acid at position 93, the amino acid at position 230, and the amino acid at position 234; or
(37) the amino acid at position 93, the amino acid at position 193, the amino acid at position 230, and the amino acid at position 234; or
(38) the amino acid at position 93, the amino acid at position 190, the amino acid at position 193, and the amino acid at position 234; or
(39) the amino acid at position 93, the amino acid at position 227, the amino acid at position 193, and the amino acid at position 234; or
(40) the amino acid at position 93, the amino acid at position 234, and the amino acid at position 373; or
(41) the amino acid at position 230, the amino acid at position 238, and the amino acid at position 373; or
(42) the amino acid at position 93, the amino acid at position 141, and the amino acid at position 234; or
(43) the amino acid at position 93, the amino acid at position 141, the amino acid at position 230, and the amino acid at position 234; or
(44) the amino acid at position 141, the amino acid at position 230, and the amino acid at position 238; or
(45) the amino acid at position 227, the amino acid at position 230, and the amino acid at position 373; or
(46) the amino acid at position 93, the amino acid at position 227, and the amino acid at position 234; or
(47) the amino acid at position 93, the amino acid at position 227, the amino acid at position 230, and the amino acid at position 234; or
(48) the amino acid at position 93, the amino acid at position 227, the amino acid at position 234, and the amino acid at position 373; or
(49) the amino acid at position 227, the amino acid at position 230, the amino acid at position 238, and the amino acid at position 373.

13. The mutant according to claim 11, **characterized in that** the amino acid mutation site further comprises the following:
(1) the amino acid at position 509; or
(2) the amino acid at position 505; or
(3) the amino acid at position 502; or
(4) the amino acid at position 501; or
(5) the amino acid at position 505 and the amino acid at position 509; or
(6) the amino acid at position 502 and the amino acid at position 509; or
(7) the amino acid at position 501 and the amino acid at position 509; or
(8) the amino acid at position 502 and the amino acid at position 505; or
(9) the amino acid at position 501 and the amino acid at position 505; or
(10) the amino acid at position 502, the amino acid at position 505, and the amino acid at position 509; or
(11) the amino acid at position 501, the amino acid at position 505, and the amino acid at position 509.

14. The mutant according to claim 12, **characterized in that** the amino acid mutation site comprises the following:
(1) the amino acid at position 92; or
(2) the amino acid at position 96; or
(3) the amino acid at position 227; or
(4) the amino acid at position 230; or
(5) the amino acid at position 238; or
(6) the amino acid at position 227 and the amino acid at position 230; or
(7) the amino acid at position 92 and the amino acid at position 227; or
(8) the amino acid at position 92 and the amino acid at position 230; or
(9) the amino acid at position 227 and the amino acid at position 238; or
(10) the amino acid at position 230 and the amino acid at position 238; or
(11) the amino acid at position 93 and the amino acid at position 234; or
(12) the amino acid at position 92 and the amino acid at position 234; or
(13) the amino acid at position 193, the amino acid at position 227, and the amino acid at position 230; or
(14) the amino acid at position 190, the amino acid at position 193, the amino acid at position 227, and the amino acid at position 230; or
(15) the amino acid at position 93, the amino acid at position 230, and the amino acid at position 234; or
(16) the amino acid at position 93, the amino acid at position 234, and the amino acid at position 373; or
(17) the amino acid at position 230, the amino acid at position 238, and the amino acid at position 373; or
(18) the amino acid at position 93, the amino acid at position 141, and the amino acid at position 234; or
(19) the amino acid at position 93, the amino acid at position 141, the amino acid at position 230, and the amino acid at position 234; or
(20) the amino acid at position 141, the amino acid at position 230, and the amino acid at position 238; or
(21) the amino acid at position 227, the amino acid at position 230, and the amino acid at position 373; or
(22) the amino acid at position 93, the amino acid at position 227, and the amino acid at position 234; or
(23) the amino acid at position 93, the amino acid at position 227, the amino acid at position 234, and the amino acid at position 373; or
(24) the amino acid at position 190, the amino acid at position 193, and the amino acid at position 230; or
(25) the amino acid at position 193 and the amino acid at position 230; or
(26) the amino acid at position 93, the amino acid at position 193, the amino acid at position 230, and the amino acid at position 234; or
(27) the amino acid at position 93, the amino acid at position 190, the amino acid at position 193, and the amino acid at position 234; or
(28) the amino acid at position 93, the amino acid at position 227, the amino acid at position 193, and the amino acid at position 234; or
(29) the amino acid at position 93, the amino acid at position 227, the amino acid at position 230, and the amino acid at position 234 ; or
(30) the amino acid at position 227, the amino acid at position 230, the amino acid at position 238, and the amino acid at position 373.

15. The mutant according to claim 13, **characterized in that** the amino acid mutation site further comprises the following:
(1) the amino acid at position 509; or
(2) the amino acid at position 505; or
(3) the amino acid at position 505 and the amino acid at position 509; or
(4) the amino acid at position 502 and the amino acid at position 509; or
(5) the amino acid at position 501 and the amino acid at position 509.

16. The mutant according to claim 1, **characterized in that** the amino acid mutation site comprises at least one of the following:
(1) the amino acid at position 79 is substituted by C, and the amino acid at position 220 is substituted by C; or
(2) the amino acid at position 82 is substituted by C, and the amino acid at position 224 is substituted by C; or
(3) the amino acid at position 92 is substituted by C, and the amino acid at position 234 is substituted by C; or
(4) the amino acid at position 93 is substituted by C, and the amino acid at position 234 is substituted by C; or
(5) the amino acid at position 82 is substituted by L; or
(6) the amino acid at position 92 is substituted by L, I, M, F, Y, or W; or
(7) the amino acid at position 96 is substituted by M or Y; or
(8) the amino acid at position 141 is substituted by M, F, Y, or W; or
(9) the amino acid at position 190 is substituted by V; or
(10) the amino acid at position 193 is substituted by M, F, Y, or W; or
(11) the amino acid at position 195 is substituted by M, F, Y, or W; or
(12) the amino acid at position 199 is substituted by M, F, Y, or W; or
(13) the amino acid at position 227 is substituted by A, V, L, I, M, S, T, or Q; or
(14) the amino acid at position 230 is substituted by F; or
(15) the amino acid at position 238 is substituted by L, I, M, F, Y, or W; or
(16) the amino acid at position 373 is substituted by M, F, or Y.

17. The mutant according to claim 4, **characterized in that** the amino acid mutation site further comprises at least one of the following:
(1) the amino acid at position 501 is substituted by V, L, I, M, F, W, Y, P, or R; or
(2) the amino acid at position 502 is substituted by V, L, I, M, F, W, Y, P, or R; or
(3) the amino acid at position 505 is substituted by M or W; or
(4) the amino acid at position 509 is substituted by G, A, V, L, I, M, F, W, P, S, T, C, Y, Q, K, R, or H; or
(5) the amino acid at position 512 is substituted by I, F, M, Y, R, or K.

18. The mutant according to claim 16, **characterized in that** the amino acid mutation site comprises the following:
(1) the amino acid at position 92 is substituted by I, M, F, Y, or W; or
(2) the amino acid at position 96 is substituted by Y; or
(3) the amino acid at position 227 is substituted by M, V, or I; or
(4) the amino acid at position 230 is substituted by F; or
(5) the amino acid at position 238 is substituted by L, I, M, or W; or
(6) the amino acid at position 227 is substituted by M or V, and the amino acid at position 230 is substituted by F; or
(7) the amino acid at position 82 is substituted by L, and the amino acid at position 227 is substituted by M; or
(8) the amino acid at position 92 is substituted by M or L, and the amino acid at position 227 is substituted by M or V; or
(9) the amino acid at position 92 is substituted by M or L, and the amino acid at position 230 is substituted by F; or
(10) the amino acid at position 96 is substituted by Y, and the amino acid at position 227 is substituted by M or V; or
(11) the amino acid at position 96 is substituted by Y, and the amino acid at position 230 is substituted by F; or
(12) the amino acid at position 96 is substituted by Y, and the amino acid at position 238 is substituted by L, I, M, or W; or
(13) the amino acid at position 89 is substituted by L, and the amino acid at position 230 is substituted by F; or
(14) the amino acid at position 227 is substituted by M, and the amino acid at position 238 is substituted by L, I, M, or W; or
(15) the amino acid at position 230 is substituted by F, and the amino acid at position 238 is substituted by L, I, M, or W; or
(16) the amino acid at position 193 is substituted by M, and the amino acid at position 195 is substituted by M, F, or Y; or
(17) the amino acid at position 190 is substituted by V, the amino acid at position 193 is substituted by M, and the amino acid at position 195 is substituted by M, F, or Y; or
(18) the amino acid at position 193 is substituted by M, and the amino acid at position 199 is substituted by M or F; or
(19) the amino acid at position 141 is substituted by M or F, and the amino acid at position 373 is substituted by M, F, or Y; or
(20) the amino acid at position 82 is substituted by C, and the amino acid at position 224 is substituted by C; or
(21) the amino acid at position 92 is substituted by C, and the amino acid at position 238 is substituted by C; or
(22) the amino acid at position 79 is substituted by C, and the amino acid at position 220 is substituted by C; or
(23) the amino acid at position 93 is substituted by C, and the amino acid at position 234 is substituted by C; or
(24) the amino acid at position 92 is substituted by C, and the amino acid at position 234 is substituted by C; or
(25) the amino acid at position 193 is substituted by M, the amino acid at position 227 is substituted by M or V, and the amino acid at position 230 is substituted by F; or
(26) the amino acid at position 190 is substituted by V, the amino acid at position 193 is substituted by M, and the amino acid at position 230 is substituted by F; or
(27) the amino acid at position 193 is substituted by M, and the amino acid at position 230 is substituted by F; or
(28) the amino acid at position 190 is substituted by V, the amino acid at position 193 is substituted by M, the amino acid at position 227 is substituted by M or V, and the amino acid at position 230 is substituted by F; or
(29) the amino acid at position 93 is substituted by C, the amino acid at position 230 is substituted by F, and the amino acid at position 234 is substituted by C; or
(30) the amino acid at position 93 is substituted by C, the amino acid at position 193 is substituted by M, the amino acid at position 230 is substituted by F, and the amino acid at position 234 is substituted by C; or
(31) the amino acid at position 93 is substituted by C, the amino acid at position 190 is substituted by V, the amino acid at position 193 is substituted by M, and the amino acid at position 234 is substituted by C; or
(32) the amino acid at position 93 is substituted by C, the amino acid at position 227 is substituted by M or V, and the amino acid at position 234 is substituted by C; or
(33) the amino acid at position 93 is substituted by C, the amino acid at position 193 is substituted by M, the amino acid at position 227 is substituted by M or V, and the amino acid at position 234 is substituted by C; or
(34) the amino acid at position 93 is substituted by C, the amino acid at position 234 is substituted by C, and the amino acid at position 373 is substituted by M, F, or Y; or
(35) the amino acid at position 230 is substituted by F, the amino acid at position 238 is substituted by L, I, M, or W, and the amino acid at position 373 is substituted by M, F, or Y; or
(36) the amino acid at position 93 is substituted by C, the amino acid at position 141 is substituted by M or F, and the amino acid at position 234 is substituted by C; or
(37) the amino acid at position 93 is substituted by C, the amino acid at position 141 is substituted by M or F, the amino acid at position 230 is substituted by F, and the amino acid at position 234 is substituted by C; or
(38) the amino acid at position 141 is substituted by M or F, the amino acid at position 230 is substituted by F, and the amino acid at position 238 is substituted by L, I, M, or W; or
(39) the amino acid at position 227 is substituted by M or V, the amino acid at position 230 is substituted by F, and the amino acid at position 373 is substituted by M, F, or Y; or
(40) the amino acid at position 93 is substituted by C, the amino acid at position 227 is substituted by M or V, the amino acid at position 230 is substituted by F, and the amino acid at position 234 is substituted by C; or
(41) the amino acid at position 93 is substituted by C, the amino acid at position 227 is substituted by M or V, the amino acid at position 234 is substituted by C, and the amino acid at position 373 is substituted by M, F or Y; or
(42) the amino acid at position 227 is substituted by M or V, the amino acid at position 230 is substituted by F, the amino acid at position 238 is substituted by L, I, M, or W, and the amino acid at position 373 is substituted by M, F or Y.

19. The mutant according to claim 17, **characterized in that** the amino acid mutation site further comprises the following:
(1) the amino acid at position 509 is substituted by F, M, or L; or
(2) the amino acid at position 505 is substituted by W; or
(3) the amino acid at position 502 is substituted by Y; or
(4) the amino acid at position 501 is substituted by M; or
(5) the amino acid at position 505 is substituted by W, and the amino acid at position 509 is substituted by F, L, or M; or
(6) the amino acid at position 502 is substituted by Y, and the amino acid at position 509 is substituted by F; or
(7) the amino acid at position 501 is substituted by M, and the amino acid at position 509 is substituted by F; or
(8) the amino acid at position 502 is substituted by Y, and the amino acid at position 505 is substituted by W; or
(9) the amino acid at position 501 is substituted by M, and the amino acid at position 505 is substituted by W; or
(10) the amino acid at position 502 is substituted by Y, the amino acid at position 505 is substituted by W, and the amino acid at position 509 is substituted by F; or
(11) the amino acid at position 501 is substituted by M, the amino acid at position 505 is substituted by W, and the amino acid at position 509 is substituted by F.

20. The mutant according to claim 18, **characterized in that** an amino acid sequence of the mutant is as follows:
(1) the amino acid at position 92 is substituted by I, M, F, Y, or W; or
(2) the amino acid at position 96 is substituted by Y; or
(3) the amino acid at position 227 is substituted by M or V; or
(4) the amino acid at position 230 is substituted by F; or
(5) the amino acid at position 238 is substituted by L, I, M, or W; or
(6) the amino acid at position 227 is substituted by M or V, and the amino acid at position 230 is substituted by F; or
(7) the amino acid at position 92 is substituted by M, and the amino acid at position 227 is substituted by M or V; or
(8) the amino acid at position 92 is substituted by M, and the amino acid at position 230 is substituted by F; or
(9) the amino acid at position 227 is substituted by M or V, and the amino acid at position 238 is substituted by L or I; or
(10) the amino acid at position 230 is substituted by F, and the amino acid at position 238 is substituted by L or I; or
(11) the amino acid at position 96 is substituted by Y, and the amino acid at position 227 is substituted by M or V; or
(12) the amino acid at position 96 is substituted by Y, and the amino acid at position 230 is substituted by F; or
(13) the amino acid at position 96 is substituted by Y, and the amino acid at position 238 is substituted by L, I, M, or W; or
(14) the amino acid at position 93 is substituted by C, and the amino acid at position 234 is substituted by C; or
(15) the amino acid at position 92 is substituted by C, and the amino acid at position 234 is substituted by C; or
(16) the amino acid at position 193 is substituted by M, the amino acid at position 227 is substituted by M or V, and the amino acid at position 230 is substituted by F; or
(17) the amino acid at position 190 is substituted by V, the amino acid at position 193 is substituted by M, the amino acid at position 227 is substituted by M or V, and the amino acid at position 230 is substituted by F; or
(18) the amino acid at position 93 is substituted by C, the amino acid at position 230 is substituted by F, and the amino acid at position 234 is substituted by C; or
(19) the amino acid at position 93 is substituted by C, the amino acid at position 234 is substituted by C, and the amino acid at position 373 is substituted by M or F; or
(20) the amino acid at position 230 is substituted by F, the amino acid at position 238 is substituted by L or I, and the amino acid at position 373 is substituted by M or F; or
(21) the amino acid at position 93 is substituted by C, the amino acid at position 141 is substituted by M or F, and the amino acid at position 234 is substituted by C; or
(22) the amino acid at position 93 is substituted by C, the amino acid at position 141 is substituted by M or F, the amino acid at position 230 is substituted by F, and the amino acid at position 234 is substituted by C; or
(23) the amino acid at position 141 is substituted by M or F, the amino acid at position 230 is substituted by F, and the amino acid at position 238 is substituted by L or I; or
(24) the amino acid at position 227 is substituted by M or V, the amino acid at position 230 is substituted by F, and the amino acid at position 373 is substituted by M or F; or
(25) the amino acid at position 93 is substituted by C, the amino acid at position 227 is substituted by M or V, and the amino acid at position 234 is substituted by C; or
(26) the amino acid at position 93 is substituted by C, the amino acid at position 227 is substituted by M or V, the amino acid at position 234 is substituted by C, and the amino acid at position 373 is substituted by M or F; or
(27) the amino acid at position 190 is substituted by V, the amino acid at position 193 is substituted by M, and the amino acid at position 230 is substituted by F; or
(28) the amino acid at position 193 is substituted by M, and the amino acid at position 230 is substituted by F; or
(29) the amino acid at position 93 is substituted by C, the amino acid at position 193 is substituted by M, the amino acid at position 230 is substituted by F, and the amino acid at position 234 is substituted by C; or
(30) the amino acid at position 93 is substituted by C, the amino acid at position 190 is substituted by V, the amino acid at position 193 is substituted by M, and the amino acid at position 234 is substituted by C; or
(31) the amino acid at position 93 is substituted by C, the amino acid at position 227 is substituted by M or V, the amino acid at position 193 is substituted by M, and the amino acid at position 234 is substituted by C; or
(32) the amino acid at position 93 is substituted by C, the amino acid at position 227 is substituted by M or V, the amino acid at position 230 is substituted by F, and the amino acid at position 234 is substituted by C; or
(33) the amino acid at position 227 is substituted by M or V, the amino acid at position 230 is substituted by F, the amino acid at position 238 is substituted by L or I, and the amino acid at position 373 is substituted by M or F.

21. The mutant according to claim 19, **characterized in that** the amino acid mutation site further comprises the following:
(1) the amino acid at position 509 is substituted by F, M, or L; or
(2) the amino acid at position 505 is substituted by W; or
(3) the amino acid at position 505 is substituted by W, and the amino acid at position 509 is substituted by F or L; or
(4) the amino acid at position 502 is substituted by Y, and the amino acid at position 509 is substituted by F; or
(5) the amino acid at position 501 is substituted by M, and the amino acid at position 509 is substituted by F.

22. The mutant according to claim 16, **characterized in that** an amino acid sequence of the mutant is a sequence shown in SEQ ID No. 35-166, or a sequence having identity greater than 90% and having same or substantially same biological functions as the sequence shown in SEQ ID No. 35-166.

23. The mutant according to claim 1, being in a trimeric form.

24. The mutant according to claim 23, **characterized in that** the trimeric form is a homotrimer form.

25. The mutant according to claim 1, **characterized in that** a C-terminus of the mutant lacks a transmembrane region and an intracellular region.

26. The mutant according to claim 1 or 25, **characterized in that** a trimerization domain is directly or indirectly linked to the C-terminus of the mutant.

27. The mutant according to claim 26, **characterized in that** the trimerization domain is a GCN4 leucine zipper, collagen or T4 foldon.

28. The mutant according to claim 26, **characterized in that** the C-terminus of the mutant is linked to the trimerization domain via a linker.

29. The mutant according to claim 28, **characterized in that** the linker is a GS repeat sequence, a GGS repeat sequence, an SAIG repeat sequence, a GSS repeat sequence or a GG repeat sequence.

30. The mutant according to claim 1, further comprising a functional tag.

31. Virus-like particles, comprising the mutant according to any one of claims 1 to 30.

32. An isolated nucleic acid molecule, encoding the mutant according to any one of claims 1 to 30.

33. A vector, comprising a sequence of the nucleic acid molecule according to claim 30 or the mutant according to any one of claims 1 to 30.

34. The vector according to claim 33, being a protein expression vector, a gene delivery vector, or a protein delivery vector.

35. The vector according to claim 34, **characterized in that** the gene delivery vector is a viral vector or a non-viral vector;
the viral vector comprises a retrovirus vector, a lentivirus vector, an adenovirus vector, an adeno-associated virus vector, a poxvirus vector, a herpes simplex virus vector or a Sendai virus vector; and the non-viral vector comprises a liposome, a cationic polymer vector, a lipid nanoparticle vector or a multifunctional envelope-type nano vector.

36. A host cell, comprising the nucleic acid molecule according to claim 32 or the vector according to any one of claims 33 to 35.

37. A protein conjugate, comprising the mutant according to any one of claims 1 to 30.

38. A pharmaceutical composition, comprising the mutant according to any one of claims 1 to 30, the isolated nucleic acid molecule according to claim 32, the vector according to any one of claims 33 to 35, the host cell according to claim 36, the protein conjugate according to claim 37, and a pharmaceutically acceptable carrier.

39. The pharmaceutical composition according to claim 38, being a vaccine.

40. The pharmaceutical composition according to claim 39, **characterized in that** the vaccine is a protein vaccine, a nucleic acid vaccine, or a vector vaccine.

41. Use of the mutant according to any one of claims 1 to 30, the virus-like particles according to claim 31, the isolated nucleic acid molecule according to claim 32, the vector according to any one of claims 33 to 35, the host cell according to claim 36, the protein conjugate according to claim 37, or the pharmaceutical composition according to any one of claims 38 to 40 for preparation of a product for preventing or treating an RSV infection.

42. Use of the mutant according to any one of claims 1 to 30, the isolated nucleic acid molecule according to claim 32, the vector according to any one of claims 33 to 35, and the host cell according to claim 36 for preparation of an antibody against a prefusion conformation of the RSV F protein.

43. A method for preventing or treating an RSV infection, **characterized in that** an effective dose of the pharmaceutical composition according to any one of claims 38 to 40 is administered to a patient.
